(19) Europäisches Patentamt
European Patent Office
Office européen des brevets

(11) Publication number: 0 040 922 A1

(12) EUROPEAN PATENT APPLICATION

(21) Application number: 81302080.7

(22) Date of filing: 11.05.81

(51) Int. Cl.³: C 12 N 15/00, C 12 P 21/02, C 07 H 21/04, C 12 N 1/20, C 07 C 103/52 // C12R1/19, C12R1/38, C12R1/07

(30) Priority: 12.05.80 GB 8015635
15.08.80 GB 8026661

(43) Date of publication of application: 02.12.81
Bulletin 81/48

(84) Designated Contracting States: AT BE CH DE FR GB IT LI LU NL SE

(71) Applicant: BIOGEN N.V., 15 Pietermaai, Willemstad Curacao, Netherlands Antilles (NL)

(72) Inventor: Nofschneider, Peter-Hans, Noerdl. Auffahrcallee 65, 19 München (DE)
Inventor: Kupper, Hans Albert, 41 Avenue Dumas, 1206 Geneva (CH)
Inventor: Schaller, Heinz, Jahnstrasse 21, 6900 Heidelberg (DE)
Inventor: Keller, Walter, Quenskestrasse 34, 6900 Heidelberg (DE)

(74) Representative: Bannerman, David Gardner et al, Withers & Rogers 4 Dyer's Buildings Holborn, London, EC1N 2JT (GB)

# (54) DNA sequences, recombinant DNA molecules and processes for producing polypeptides with the specificity of foot and mouth disease viral antigens.

(57) DNA sequences, recombinant DNA molecules and hosts transformed with them which produce antigenic polypeptides of FMDV and replicate virus specific nucleotide sequences coding therefor and methods of making and using these molecules, hosts, DNA sequences and antigenic polypeptides. The DNA sequences and recombinant DNA molecules of this invention are characterized by DNA inserts that code for at least one FMDV antigenic polypeptide. In hosts capable of expressing these polypeptides, these DNA sequences and recombinant DNA molecules permit the replication and identification of virus specific nucleotide sequences and the production and identification of antigenic polypeptides and their use in compositions and methods for rendering animals resistant to FMDV at least for some period of time.

EP 0 040 922 A1

# DNA SEQUENCES, RECOMBINANT DNA MOLECULES AND PROCESSES FOR PRODUCING POLYPEPTIDES WITH THE SPECIFICITY OF FOOT AND MOUTH DISEASE VIRAL ANTIGENS

## TECHNICAL FIELD OF INVENTION

This invention relates to DNA sequences, recombinant DNA molecules and processes for producing polypeptides with the specificity of foot and mouth disease ("FMD") viral antigens. More particularly, the invention relates to DNA sequences and recombinant DNA molecules expressed in appropriate host organisms. The DNA sequences and recombinant DNA molecules disclosed herein are characterized in that they code or include fragments that code for antigenic polypeptides of foot and mouth disease virus ("FMDV") or polypeptides with the specificity of foot and mouth disease viral antigens. As will be appreciated from the disclosure to follow, the DNA sequences, recombinant DNA molecules and processes of this invention may be used in the production of antigenic polypeptides useful in compositions and methods for rendering animals resistant to FMDV for at least some period of time.

## BACKGROUND ART

FMD virus is a picornavirus which affects cloven-footed animals. It is one of the most infectious and easily transmitted diseases of livestock. The disease is generally characterized by vesicular lesions on the feet and mouth of the infected animal. Deterioration in body condition generally lowers production of animal products by 25%. Epizootics of FMDV cause major economic losses in the production and marketing of meat, dairy products, wool, hides and other commodities. For example, during the epizootic in Great Britain in 1967-68 nearly 500,000 infected or exposed cattle, swine, sheep and goats were destroyed in the process of eradicating the disease.

There are seven distinct serotypes of FMD virus. These are the European or classical types O, A and C, the Southern African Territories types SAT1, SAT2 and SAT3 and the Asian type I. E.g., K.J.H. Robson et al., "An Assessment By Competition Hybridization Of The Sequence Homology Between The RNAs Of The Seven Serotypes Of FMDV", J. Gen. Virol., 37, pp. 271-76 (1977). Types O, A and C have been found in Europe, South America, Asia and the northern part of Africa, although the range is extending. The three Southern African Territories types were first detected in Southern Africa, but again the disease type is spreading in geographic location. The Asian type occurs in Asian countries from the Eastern Mediterranean to the Far East. Each of these serotypes may also be divided serologically into several subtypes.

Vaccines to protect animals from FMDV are available. Most commonly these vaccines comprise inactivated or attenuated whole virus. They are administered under known schedules and regimes on an annual or sometimes quarter-annual basis. Because the seven virus types display a lack of cross-immunity (Robson et al., supra) and different areas of the world have a different spectra of virus types, vaccination is sometimes carried out with a bivalent or trivalent material. However, this is not always necessary or advisable.

Production of FMD whole virus vaccines is beset by several major problems. First, because of the infectious character of the virus, laboratory growth of virus for vaccines must be done in isolated facilities under high containment. Second, the virus-based vaccines often display an unacceptable variation in potency after production and inactivation. Third, the vaccines must be tested under very controlled conditions to insure proper efficiency of attenuation or inactivation. Otherwise, the vaccine may cause accidental active infection or subacute progressive disease in the treated animals. All of these production problems result in higher costs for the ultimate vaccine. In addition to the above described production problems, the use of whole-virus vaccines results in a small but significant number of allergic side reactions in the treated animals. These undesirable side effects are probably caused by the many irrelevant antigenic determinants of the viral and non-viral proteins that usually contaminate viral vaccines.

One approach to avoid some of the problems inherent in the production and use of whole virus vaccines is to employ viral subunit vaccines comprising the capsid proteins of FMD virus. E.g., U.S. patent 4,140,763; H. L. Bachrach et al., "An Experimental Protein Vaccine For Foot-And-Mouth Disease", in Perspectives in Virology, (ed. M. Pollard), vol. 10, chap. 10, pp. 147-159 (1978).

FMD virus contains primarily four capsid proteins, identified as VP1, VP2, VP3 and VP4. The capsid proteins of FMD virus collectively protect the viral ribonucleic acid ("RNA") core against various inactivating agents. The neutralizing antigen of FMDV seems to be embodied in the VP1 polypeptide (VP3 in United States terminology) (H. L. Bachrach et al., "An Experimental Subunit Vaccine For Foot-And-Mouth Disease", International Symposium On Foot-And-Mouth Disease, Lyon 1976, Develop. Biol. Standard, 35, pp. 155-160 (S.

Karger, Basel 1977)).* This polypeptide has been purified and employed to vaccinate swine against challenges by virus (H. L. Bachrach et al., supra). However, at least 10 times more protein than virus-based vaccines were required to effect immunization. Therefore, two or more vaccinations with the VP1 protein-based subunit virus are usually required to protect an animal from FMD virus.

The use of these subunit vaccines eliminate antibody formation against the many irrelevant antigenic determinants of the viral and non-viral proteins that contaminate viral vaccines. Their use may therefore eliminate the side effects of viral vaccines. Further, the subunit vaccines are devoid of viral nucleic acid and therefore presumably without risk of causing active infection or subacute progressive disease in the treated herds. However, while these subunit vaccines avoid some of the problems which characterize whole virus based vaccines, there are also disadvantages in their use. First, to obtain the capsid protein, virus must be cultured and grown. Therefore, the isolated facilities and high production containment attendant to FMD virus growth are not avoided. Second, the proteins must be separated from the virus and highly purified. This process is both slow and expensive. Moreover, if the proteins are not sufficiently purified, the resultant vaccine may still contain enough virus to cause accidental infection or subacute disease.

Recent advances in molecular biology have made it possible to introduce the DNA coding for specific non-bacterial proteins into bacterial cells. In general,

---

* Moreover, the antigenic portion of that protein appears to reside in the last 1/3 of the protein, i.e., nearest its COOH terminus (R. Franz et al., "Localization And Characterization Of Two Immunogenic Regions On The Coat Protein $VP_{Thr}$ Of Foot-And-Mouth Disease Virus (FMDV) Subtype $O_1K$ Inducing Neutralizing Antibodies, Munich (December 1980)).

with DNA, other than that prepared via chemical synthesis, the construction of such recombinant DNA molecules comprises the steps of producing a single-stranded DNA copy (cDNA) of a messenger RNA (mRNA) template for the desired protein; converting the cDNA to double-stranded DNA; linking the DNA to an appropriate site in an appropriate cloning vehicle to form a recombinant molecule and transforming an appropriate host with that recombinant DNA molecule. Such transformation may permit the host to produce the desired protein.

Several non-bacterial genes and proteins have been obtained in bacterial hosts using recombinant DNA technology. These include, for example, leukocyte interferon (S. Nagata et al., "Synthesis In E. coli Of A Polypeptide With Human Leukocyte Interferon Activity", Nature, 284, pp. 316-20 (1980)), antigens of human hepatitis B virus (C. J. Burrell et al., "Expression In Escherichia coli: Of Hepatitis B Virus DNA Sequences Cloned In Plasmid pBR322", Nature, 279, pp. 43-7 (1979) and M. Pasek et al., "Hepatitis B Virus Genes And Their Expression In E. coli", Nature, 282, pp. 575-79 (1979)), and SV40t antigen (T. M. Roberts et al., "Synthesis Of Simian Virus 40t Antigen In Escherichia coli", Proc. Natl. Acad. Sci. USA, 76, pp. 5596-600 (1979)).

Prior recombinant schemes have been limited to making cDNA copies of mRNA of 800-900 nucleotides (leukocyte interferon) or mRNA of 2000-3000 nucleotides (ovalbumin or RNA tumors) or have used DNA already available in nature (hepatitis B virus, Dane particle DNA; SV40t antigen, SV40 DNA).

## DISCLOSURE OF THE INVENTION

The present invention generally solves the problems referred to above by providing at least one DNA sequence coding for a polypeptide displaying FMDV antigenicity. More particularly, we provide in accordance with the invention a DNA sequence characterized in that at least a portion thereof codes for a polypeptide displaying FMDV antigenicity and being selected from the group consisting of (a) FMDV-715, FMDV-144, FMDV-1034, FMDV-1448, FMDV-1824, FMDV-1933, (b) DNA sequences which hybridize to any of the foregoing DNA sequences, (c) DNA sequences, from whatever source obtained, including natural, synthetic or semi-synthetic sources, related by mutation, including single or multiple, base substitutions, deletions, insertions and inversions to any of the foregoing DNA sequences, and (d) DNA sequences comprising sequences of codons which code for an antigenic polypeptide containing an amino acid sequence similar to those coded for by codons of any of the foregoing DNA sequences.

The DNA sequences of the invention are such so as to enable synthesis of a protein from a genome of about 7,700 bases, which apparently is translated entirely and without interruption to produce a total translation product or "polyprotein", the polyprotein then being further processed *in vivo* to produce a capsid and other FMD viral proteins. These DNA sequences also characterize the recombinant DNA molecules and processes of this invention.

By virtue of this invention, it is possible to obtain FMD viral specific nucleotide sequences and FMDV antigenic polypeptides in substantial quantities. The DNA sequences and recombinant DNA molecules of this invention permit the replication of FMD viral specific nucleotide sequences and the production of viral antigens against FMDV without the necessity of growing large amounts of virus, purifying proteins from the virus or inactivating or attenuating the virus. These DNA sequences and recombinant DNA molecules and the processes of this invention therefore avoid the problems which beset the other known methods of FMDV vaccine production and the vaccines produced thereby.

As will be appreciated from the disclosure to follow, the DNA sequences and recombinant DNA molecules of this invention are capable in an appropriate host of directing the production of antigenic polypeptides of FMDV. Replication of these DNA sequences and recombinant DNA molecules in an appropriate host also permits the production in large quantities of FMD viral specific nucleotide sequences and polypeptides without the danger of accidental spread of FMDV. The molecular structure and properties of these antigenic polypeptides and viral specific nucleotide sequences may then be determined safely without the isolation conditions required for the more usual FMDV analysis. The antigenic polypeptides and DNA sequences of this invention are useful, either as produced in the host or after appropriate derivatization or modification, in compositions and methods for detecting and improving the production of these products themselves and for use in vaccines and other immunogenic compositions and methods of treatment for FMD virus.

## BRIEF DESCRIPTION OF THE DRAWINGS

Figure 1 is a simplified schematic of the FMD virus genome, the "polyprotein" translation product and the resultant capsid and other proteins. The locations of the various proteins depicted are not absolute in relation to the FMDV genome, because they are based on relative size data alone.

Figure 2 is a schematic outline of one embodiment of a process of this invention for producing polypeptides displaying FMDV antigenicity.

Figure 3 displays the restriction map of cDNA prepared in accordance with this invention and its alignment from the 3' end of the FMDV genome. Figure 3 also displays the alignment to the cDNA, the orientation and the restriction pattern of the DNA inserts of various hybrid plasmids prepared in accordance with this invention.

Figure 4 displays the relevant segment of a restriction map of the DNA inserts of two recombinant DNA molecules prepared in accordance with this invention and the sequencing strategy used in determining the partial nucleotide sequence of them.

Figure 5 displays the relevant segment of a nucleotide sequence of one recombinant DNA molecule of this invention and its corresponding amino acid sequence.

Figure 6 is a schematic description of plasmid pPLc24 and plasmid pFMDV-1034.

Figure 7 is a schematic outline of a process for constructing another embodiment of a recombinant DNA molecule of this invention.

Figure 8 displays the FMDV genome and the restriction pattern of DNA insert FMDV-1034 and the sequencing strategy used in determining its nucleotide sequence.

Figures 9-10 display a part of the nucleotide sequence of DNA insert FMDV-1034, the nucleotide sequence for the structural gene for VP1, the nucleotide sequence of the DNA insert of one embodiment of a recombinant DNA molecule of this invention and the amino acid sequences of the polypeptides coded for by these nucleotide sequences.

Figure 11 displays a part of the nucleotide sequence of DNA insert FMDV-1448, the nucleotide sequence for the structural gene of VP3, the nucleotide sequence of a portion of a DNA insert of one embodiment of a recombinant DNA molecule of this invention and the amino acid sequences of the polypeptides coded for by these nucleotide sequences.

Figure 12 displays a schematic outline of a process for selecting and constructing another embodiment of a DNA sequence of this invention.

## BEST MODE OF CARRYING OUT THE INVENTION

In order that the invention herein described may be more fully understood, the following detailed description is set forth.

In the description the following terms are employed:

Nucleotide--A monomeric unit of DNA or RNA consisting of a sugar moiety (pentose), a phosphate, and a nitrogenous heterocyclic base. The base is linked to the sugar moiety via the glycosidic carbon (1' carbon of the pentose) and that combination of base and sugar is called a nucleoside. The base characterizes the nucleotide. The four DNA bases are adenine ("A"), guanine ("G"), cytosine ("C"), and thymine ("T"). The four RNA bases are A, G, C and uracil ("U").

DNA Sequence--A linear array of nucleotides connected one to the other by phosphodiester bonds between the 3' and 5' carbons of adjacent pentoses.

Codon--A DNA sequence of three nucleotides (a triplet) which encodes through mRNA an amino acid, a translation start signal or a translation termination signal. For example, the nucleotide triplets TTA, TTG, CTT, CTC, CTA and CTG encode for the amino acid leucine ("Leu"), TAG, TAA and TGA are translation stop signals and ATG is a translation start signal.

Reading Frame--The grouping of codons during translation of mRNA into amino acid sequences. During translation the proper reading frame must be maintained. For example, the sequence GCTGGTTGTAAG may be translated in three reading frames or phases, each of which affords a different amino acid sequence

GCT GGT TGT AAG--Ala-Gly-Cys-Lys
G CTG GTT GTA AG--Leu-Val-Val
GC TGG TTG TAA G--Trp-Leu-(STOP)

<u>Polypeptide</u>--A linear array of amino acids connected one to the other by peptide bonds between the α-amino and carboxy groups of adjacent amino acids.

<u>Genome</u>--The entire DNA of a cell or a virus. It includes <u>inter alia</u> the structural genes coding for the polypeptides of the cell or virus, as well as its operator, promoter and ribosome binding and interaction sequences, including sequences such as the Shine-Dalgarno sequences.

<u>Structural Gene</u>--A DNA sequence which encodes through its template or messenger RNA ("mRNA") a sequence of amino acids characteristic of a specific polypeptide.

<u>Transcription</u>--The process of producing mRNA from a structural gene.

<u>Translation</u>--The process of producing a polypeptide from mRNA.

<u>Expression</u>--The process undergone by a structural gene to produce a polypeptide. It is a combination of transcription and translation.

<u>Plasmid</u>--A non-chromosomal double-stranded DNA sequence comprising an intact "replicon" such that the plasmid is replicated in a host cell. When the plasmid is placed within a unicellular organism, the characteristics of that organism may be changed or transformed as a result of the DNA of the plasmid. For example, a plasmid carrying the gene for tetracycline resistance ($Tet^R$) transforms a cell previously sensitive to tetracycline into one which is resistant to it. A cell transformed by a plasmid is called a "transformant".

<u>Phage or Bacteriophage</u>--Bacterial virus, many of which consist of DNA sequences encapsidated in a protein envelope or coat ("capsid protein").

<u>Cloning Vehicle</u>--A plasmid, phage DNA or other DNA sequence which is able to replicate in a host cell, which is characterized by one or a small number of endonuclease recognition sites at which such DNA sequences may be cut in a determinable fashion without attendant loss of an essential biological function of the DNA, e.g., replication, production of coat proteins or loss of promoter or binding sites, and which contains a marker suitable for use in the identification of transformed cells, e.g., tetracycline resistance or ampicillin resistance. A cloning vehicle is often called a vector.

Cloning--The process of obtaining a population of organisms or DNA sequences derived from one such organism or sequence by asexual reproduction.

Recombinant DNA Molecule or Hybrid DNA--A molecule consisting of segments of DNA from different genomes which have been joined end-to-end outside of living cells and have the capacity to infect some host cell and be maintained therein.

Expression Control Sequence--A sequence of nucleotides that controls and regulates expression of structural genes when operatively linked to those genes. They include the lac system, the trp system, major operator and promoter regions of phage λ, the control region of fd coat protein and other sequences known to control the expression of genes of prokaryotic or eukaryotic cells or their viruses.

Referring now to Figure 1, we have shown therein a simplified diagram of an FMDV genome. This genome of single stranded RNA encodes all of the genetic information of the FMD virus. The genome consists of about 7,700 bases or about 2.8 x $10^6$ daltons. The virion RNA is the plus strand of the virus and is poly A terminated at its 3' end and has a small protein (VPG) covalently attached to its 5' terminus.

It has been demonstrated in vivo that only one major initiation site for protein synthesis exists in this genome. The genome is therefore probably translated entirely and without interruption to produce a total translation product or "polyprotein" (Figure 1) (T. R. Doel et al., "A Re-appraisal Of The Biochemical Map Of Foot-And-Mouth Disease Virus RNA", J. Gen. Virol., 41, pp. 395-404 (1978)). This polyprotein is then apparently further processed in vivo to produce the three primary products P 100 (122), P 55 and P 88 of FMDV. One of these primary products P 88 has been reported to be the precursor of the four capsid proteins VP1, VP2, VP3 and VP4 (Figure 1) of the virus (T. R. Doel et al., supra). Therefore, the four capsid proteins apparently arise from a single region of the genome. However, the actual structure or location of this region of the genome (about 2400 base pairs) has not been determined. Nor have the complete structures of the capsid proteins been determined. For example, only the first 40 amino acids from the amino terminal end of VP1 have been reported--Thr-Thr-Ser(?)-Ala-Gly-Glu-Lys(?)-Ala-Asp-Pro-Val-Thr-Thr-Thr-Val-Glu-Asn-Tyr-Gly-Gly-Glu-Thr-Gln-Ile-Gln-Arg-Arg-Gln-His(?)-Thr(?)-Asp-Val-Trp(?)-Phe-Ile-Met-Asp-(?)-Phe-Val--(K. Strohmaier et al., "The N-Terminal Sequence Of Three Coat Proteins Of Foot-And-Mouth Disease Virus", Biochemical & Biophysical Research Comm. ("BRCC"), 85, pp. 1640-45 (1978)).*

In order that this invention may be more fully understood the following example of one embodiment thereof is included.

## EXAMPLE

The RNA used in this embodiment of the invention was extracted from cultures of FMDV, type O, substantially as described by J.F.E. Neuman et al., "Purification And Identification Of The RNA-Dependent

---

* "(?)" denotes an amino acid determination which is not definite.

RNA Polymerase Of Foot-And-Mouth Disease Virus", J. Gen. Virol., 45, pp. 497-507 (1979).

FMDV strain O (Kaufbeuren) was grown in BHK cell monolayers in several roller bottles. The virus was collected from the maintenance medium by addition of polyethylene glycol 6000 (to 8%) and precipitation. The virus was purified by banding in 1-1.44 g/$cm^3$ gradients of cesium chloride substantially as described by K. Strohmaier and K. H. Adams, Zbl. Vet. Med., B23, pp. 483-506 (1976). After dialysis, the virus was treated with proteinase as described by M. J. Grubman et al., Virology, 97, p. 22 et seq. (1979) and the RNA purified by two cycles of centrifugation in a 15-30% sucrose gradient. The RNA was then precipitated from the peak fractions with ethanol and redissolved in water. The resulting viral RNA preparation (0.78 mg/ml) had a 34S sedimentation coefficient (measured with rRNA as marker) and $OD_{260}/OD_{280} = 2$.

Similar techniques are employed in preparing RNA from FMDV of other serotypes. The produced RNA is then treated as described below to produce the desired recombinant DNA molecules. These molecules in hosts capable of expression are employed to produce polypeptides which display FMDV antigenicity and to replicate virus specific nucleotide sequences.

## SYNTHESIS OF DOUBLE-STRANDED FMDV-DNA

FMDV-RNA prepared above was used as a template to prepare complementary FMDV-DNA ("FMDV-cDNA"), essentially as described by M. P. Wickens, et al., "Synthesis Of Double-Stranded DNA Complementary To Lysozyme, Ovomucoid And Ovalbumin mRNAs", J. Biol. Chem., 253, pp. 2483-495 (1978).

Single-stranded cDNA was prepared by RNA-dependent DNA polymerase (80 units) from avian myeloblastosis virus ("AMV") reverse transcriptase, initiated by a $pT_{12-18}$ primer (1 unit/ml, Collaborative Research) in

100 µl 50 mM Tris-HCl (pH 8.3), 8 mM $MgCl_2$, 25 mM β-mercaptoethanol, dATP, dCTP, dGTP and dTTP (each at 0.5 mM) and 100 µCi $^3$H-TTP (NEN), the RNA (14 µg) having been pretreated with 40 µl 2.5 mM $CH_3HgOH$ for 3 min at room temperature.

The mixture was incubated for 5 min at room temperature, then for 60 min at 42°C. Aliquots (1 µl) were taken every 20 min to monitor the kinetics of the reaction. Following incubation and boiling in a water bath for 3 min to dissociate the cDNA-RNA hybrid, the reaction mixture was quickly cooled in ethanol/carbon dioxide and placed on ice.

The cDNA strand was rendered double-stranded by DNA polymerase I by combining it with 50 µCi each of the four $^{32}$P-deoxytriphosphates, 100 µl 0.2 M HEPES buffer ((N-2-hydroxyethylpiperazine-N'-2-ethanesulfonic acid; Sigma) pH 6.9), 90 mM KCl, 0.4 mM each dATP, dCTP, dGTP and dTTP and 50 units DNA polymerase I (M. P. Wickens et al., supra). The mixture was incubated at 15°C for 2 h. Aliquots were again taken every 30 min to monitor the reaction. To stop the reaction, the mixture was extracted with phenol and passed through a G-150 Sephadex column which was equilibrated with 0.1 M NaCl, 10 mM Tris-HCl buffer (pH 7.5) and 1 mM EDTA. To remove the radioactive precursors, fractions containing the cDNA were pooled and precipitated with ethanol.

The single-stranded hairpin loop which remains on the double stranded cDNA structure, was opened by dissolving the precipitated cDNA in 675 µl Sl buffer (0.3 M NaCl, 0.03 M sodium acetate (pH 4.5) and 3 mM $ZnCl_2$). Sl enzyme (15 µl, 0.6 Vogt units/µl) was added and the mixture incubated at 37°C for 130 min. After addition of 30 µl 0.2 M EDTA, the mixture was extracted twice with phenol and precipitated as before with ethanol.

The cDNA prepared above was sized on a 1% agarose gel using appropriate markers. More than 40% of the cDNA was between 4500 and 8000 bp. The remainder of the cDNA was shorter. The cDNA was also cut with various restriction enzymes using standard procedures. The sharpness of the restriction pattern for the cDNA indicated the high molecular weight of the cDNA.

On the basis of these restriction patterns, the cDNA was aligned with the 3' end of the genome for FMDV (Figure 3). This pattern and alignment was subsequently utilized to determine the position in the FMDV genome of the DNA inserts in various hybrid plasmids prepared in accordance with this invention. A correlation of the position of these inserts in the genome and the predicted position in the genome of the coding sequences for the several proteins of FMDV permits prediction of which FMDV proteins are coded for by the DNA insert of a particular hybrid plasmid.

CLONING OF DOUBLE-STRANDED FMDV-cDNA

A wide variety of host/cloning vehicle combinations may be employed in cloning the double-stranded cDNA prepared in accordance with this invention. For example, useful cloning vehicles may consist of segments of chromosomal, non-chromosomal and synthetic DNA sequences, such as various known derivatives of SV40 and known bacterial plasmids, e.g., plasmids from E. coli including col E1, pCR1, pBR322, pMB89 and their derivatives, wider host range plasmids, e.g., RP4, and phage DNAs, e.g., the numerous derivatives of phage , e.g., NM989, and other DNA phages, e.g., M13 and Filamenteous single-stranded DNA phages and vectors derived from combinations of plasmids and phage DNAs such as plasmids which have been modified to employ phage DNA or other expression control sequences or yeast plasmids such as the 2 μ plasmid or derivatives

thereof. Useful hosts may include bacterial hosts such as strains of _E. coli_, such as _E. coli_ HB 101, _E. coli_ X1776, _E. coli_ X2282, _E. coli_ MRCI and strains of _Pseudomonas_, _Bacillus subtilis_, _Bacillus stearothermophilus_ and other _E. coli_, bacilli, yeasts and other fungi, animal or plant hosts such as animal (including human) or plant cells in culture or other hosts. Of course, not all host/vector combinations may be equally efficient. The particular selection of host/cloning vehicle combination may be made by those of skill in the art after due consideration of the principles set forth without departing from the scope of this invention.

Furthermore, within each specific cloning vehicle, various sites may be selected for insertion of the double-stranded DNA. These sites are usually designated by the restriction endonuclease which cuts them. For example, in pBR322 the _Pst_I site is located in the gene for β-lactamase, between the nucleotide triplets that code for amino acids 181 and 182 of that protein. This site was employed by S. Nagata _et al._, _supra_, in their synthesis of polypeptides displaying an immunological or biological activity of leukocyte interferon. One of the two _Hind_II endonuclease recognition sites is between the triplets coding for amino acids 101 and 102 and one of the several _Taq_ sites at the triplet coding for amino acid 45 of β-lactamase in pBR322. In similar fashion, the _Eco_RI site and the _Pvu_II site in this plasmid lie outside of any coding region, the _Eco_RI site being located between the genes coding for resistance to tetracycline and ampicillin, respectively. These sites are well recognized by those of skill in the art. It is, of course, to be understood that a cloning vehicle useful in this invention need not have a restriction endonuclease site for insertion of the chosen DNA fragment. Instead, the vehicle could be cut and joined to the fragment by alternative means.

The vector or cloning vehicle and in particular the site chosen therein for attachment of a selected DNA fragment to form a recombinant DNA molecule is determined by a variety of factors, e.g., the number of sites susceptible to a particular restriction enzyme, the size of the protein to be expressed, the susceptibility of the desired protein to proteolytic degradation by host cell enzymes, the contamination of the protein to be expressed by host cell proteins difficult to remove during purification, the expression characteristics, such as the location of start and stop codons relative to the vector sequences, and other factors recognized by those of skill in the art. The choice of a vector and an insertion site for a particular gene is determined by a balance of these factors, not all selections being equally effective for a given case.

Although several methods are known in the art for inserting foreign DNA into a cloning vehicle or vector to form a recombinant DNA molecule, the initial cloning method preferred in accordance with this invention is characterized by digesting the plasmid (in particular pBR322) with that restriction enzyme specific to the site chosen for the insertion (in particular PstI) and adding dG tails to the 3' termini by terminal transferase. In similar fashion, the double-stranded cDNA is elongated by the addition of dC tails to the 3' termini to allow joining to the tailed plasmid. The tailed plasmid and cDNA are then annealed to insert the cDNA into the appropriate site of the plasmid and to circularize the hybrid DNA, the complementary character of the tails permitting their cohesion (Figure 2). The resulting recombinant DNA molecule now carries an inserted gene at the chosen PstI restriction site (Figure 2). This method of dG-dC tailing for insertion of a foreign DNA sequence into a cloning vehicle is described by L. Villa-Komaroff et al., "A Bacterial Clone Synthesizing Proinsulin", Proc. Natl. Acad. Sci. USA, 75, pp. 3727-31 (1978).

Of course, other known methods of inserting DNA sequences into cloning vehicles to form recombinant DNA molecules may be equally useful in this invention. These include, for example, dA-dT tailing, direct ligation, synthetic linkers, exonuclease and polymerase-linked repair reactions followed by ligation, or extension of the DNA strand with DNA polymerase and an appropriate single-stranded template followed by ligation.

It should also be understood that the nucleotide sequences or cDNA fragments inserted at the selected site of the cloning vehicle may include nucleotides which are not part of the actual structural gene for the desired polypeptide or mature protein or may include only a fragment of the complete structural gene for the desired protein or mature protein. It is only important that the recombinant DNA molecule directs the production of a polypeptide having the specificity of FMD viral antigens in appropriate hosts.

The cloning vehicle or vector containing the foreign gene is employed to transform an appropriate host so as to permit that host to replicate the foreign gene and to express the protein coded by the foreign gene or portion thereof. The selection of an appropriate host is also controlled by a number of factors recognized by the art. These include, for example, the compatibility with the chosen vector, the toxicity of proteins encoded by the hybrid plasmid, the ease of recovery of the desired protein, the expression characteristics, biosafety and costs. A balance of these factors must be struck with the understanding that not all hosts may be equally effective for expression of a particular recombinant DNA molecule.

In the present synthesis, the preferred initial cloning vehicle is the bacterial plasmid pBR322 and the preferred initial restriction endonuclease site therein is the *PstI* site (Figure 2). The plasmid is a small (molecular weight approx. 2.6 megadaltons) plasmid carrying resistance genes to the antibiotics ampicillin (Amp) and tetracycline (Tet). The plasmid has been fully characterized (F. Bolivar, *et al.*, "Construction And Characterization Of New Cloning Vehicles II. A Multi-Purpose Cloning System", *Gene*, pp. 95-113 (1977); J. G. Sutcliffe, "pBR322 Restriction Map Derived From The DNA Sequence: Accurate DNA Size Markers Up To 4361 Nucleotide Pairs Long", *Nucleic Acids Research*, 5, pp. 2721-28 (1978); J. G. Sutcliffe, "Complete Nucleotide Sequence Of The *Escherichia coli* Plasmid pBR322", *Cold Spring Harbor Symposium*, 43, I, pp. 77-90 (1978)). Insertion of the cDNA product at this site provides a bacterial clone which contains a DNA sequence coding for an FMDV specific protein.

1. Preparation Of PstI-Cleaved dG-elongated pBR322

Plasmid pBR322 was digested completely at 37°C with *PstI* endonuclease (Boehringer) in 10 mM Tris-HCl (pH 7.6), 7 mM $MgCl_2$, 7mM 2-mercaptoethanol, 50 mM NaCl. The mixture was extracted with 1 vol phenol and precipitated with 2.5 vol ethanol: 0.2 M sodium acetate solution. The precipitate was washed twice with 70% ethanol and dissolved in TE buffer (10 mM Tris-HCl (pH 8.0), 1 mM EDTA).

Addition of homopolymeric dG tails by terminal deoxynucleotidyl transferase (TdT) (BRL, 6 units/µl) was done in a 50 µl reaction volume containing 1 µg of cut plasmid, 200 mM Na-K-cacodylate (pH 7.2), 1 mM $CoCl_2$, 1 mM β-mercaptoethanol, 200 mM dGTP and 12 units of TdT. Incubation was for 60 min at 37°C.

2. Preparation of dC-elongated cDNA

Approximately 1 pmole of double stranded cDNA was elongated with dCTP residues by terminal deoxynucleotidyl transferase in a 50 μl reaction volume containing 200 mM Na-K-cacodylate (pH 7.2) 1 mM $CoCl_2$, 1 mM β-mercaptoethanol, 100 mM dCTP and 15 units of TdT (BRL, 6 units/μl). Incubation was for 5 min at 37°C, and the reaction was stopped by freezing.

3. Preparation of $Ca^{++}$-Treated E. coli HB 101

$Ca^{++}$ -treated E. coli HB101 was prepared by the method of E. M. Lederberg and S. N. Cohen, "Transformation Of Salmonella Typhimurium By Plasmid Deoxyribonucleic Acid", J. Bacteriol., 119, pp. 1072-74 (1974) by inoculating E. coli HB101 in 250 ml LB medium (10 parts bactotryptone, 5 parts yeast extract and 5 parts NaCl per liter) and cultures grown at 37°C to $O.D._{650}$ = 0.65. The fresh cultures were chilled in ice water for 5 min and pelleted at 6000 rpm for 10 min at 4°C. The pellets were then resuspended in 100 ml ice-cold 0.1 M $CaCl_2$ and maintained at 0°C for 15 min and centrifuged as before. The pellets were resuspended in 100 ml 0.1 M $CaCl_2$, as before, and centrifuged prior to taking them up in 25 ml 0.1 M $CaCl_2$, supplemented with 2.5 ml glycerol. The culture suspension was divided in 2 ml aliquots and frozen in liquid $N_2$ and stored at -80°C without loss of activity (D. A. Morrison, "Transformation In Escherichia coli: Cryogenic Preservation Of Competent Cells", J. Bacteriol., 132, pp. 349-51 (1977)).

4. Annealing of dG-elongated pBR322 and dC-elongated DNA

The vector's and cDNA's complementary dG- and dC-tails permit annealing to recircularize the desired hybrid plasmid or recombinant DNA molecule. For this purpose, 20 ng (0.01 pmol) dG-tailed PstI-cleaved pBR322 vector and 0.01 pmol dC-tailed double stranded cDNA were mixed in 30 μl 1 x SSC buffer (0.15 M NaCl, 0.015 M sodium citrate). The mixture was placed in a water bath at 69°C and allowed to cool overnight to 35°C.

5. Transformation Of E. coli HB101 With The Annealed Hybrid Plasmids

L3 containment facilities were used for the transformation process and as required in all subsequent steps. Aliquots (15 μl) of the recombinant DNA molecule mixture prepared above were incubated for 2 h at 0°C with 200 μl $Ca^{++}$-treated *E. coli* HB101. The cultures were heat-shocked at 37°C for 4 min and 0.4 ml TMg medium (10 g Bactotryptone, 5 g NaCl, 2 g $MgCl_2$ per liter) added and the bacterial suspension plated directly onto agar plates containing 10 g bactotryptone, 5 g yeast extract, 5 g NaCl and 14 g bactoagar per liter supplemented with 10 μg/ml tetracycline. The plates were incubated for 2 days at 37°C.

Because plasmid pBR322 includes the gene coding for tetracycline resistance, *E. coli* hosts which have been transformed with a plasmid having that gene intact will grow in medium containing that antibotic to the exclusion of those bacteria not so transformed. Therefore, growth in tetracycline-containing medium permits selection of hosts transformed with a recombinant DNA molecule or recircularized cloning vector containing the hybrid gene.

The tetracycline resistant hosts were also screened for ampicillin resistance by repicking clones onto plates containing medium supplemented with ampicillin. Although plasmid pBR322 includes the gene coding for ampicillin resistance, plasmids which have inserts at the *PstI* site in this gene no longer render bacteria ampicillin resistant. Therefore, hosts which are resistant to tetracycline but which are sensitive to ampicillin contain plasmids having inserts in the gene for ampicillin. Here, about 87% of the colonies were resistant to tetracycline and sensitive to ampicillin. The remainder of the tetracycline resistant colonies probably were transformed with plasmids that had been recircularized without hybrid genes.

SCREENING FOR A CLONE CONTAINING FMDV cDNA

Four-hundred of the ampicillin sensitive, tetracycline resistant clones were transferred to the wells of micro-titer plates. This library of clones was subdivided into 8 groups of 50 clones (identified by numbers 101-150 through 801-850 respectively). Replicas of each set were prepared on nitrocellulose filter-layered tryptone-agar plates, supplemented with 10 μg/ml tetracycline. The plates were incubated overnight at 37°C. The nitrocellulose filters were then used for colony hybridization substantially as described by M. Grunstein and D. S. Hogness, "Colony Hybridization: A Method For The Isolation Of Cloned DNAs That Contain A Specific Gene", Proc. Natl. Acad. Sci. USA, 72, pp. 3961-65 (1975). A $^{32}P$-labelled total FMD virus RNA probe was prepared by labelling the RNA isolated previously by polynucleotide kinase ("PNK") in the presence of $\gamma^{32}P$-ATP. The fragments, which were produced by the small amounts of ribonuclease that contaminated the PNK, had an average length of 300 bases. Hybridization to this probe was carried out substantially as described by N. Maniatis et al., "The Isolation Of Structural Genes From Libraries Of Eucaryotic DNA", Cell, 15, pp. 687-701 (1978). Autoradiography of the filters revealed the presence of colonies that contained DNA sequences complementary to authentic FMD virus RNA. Colonies which displayed strong hybridization to the $^{32}P$-labelled probe were selected for further analysis.

## FURTHER ANALYSIS OF CLONES CONTAINING INSERTS HYBRIDIZING TO FMDV DNA

Twenty ml cultures of the above selected strongly hybridizing clones were prepared at 37°C in bactotryptone medium, supplemented as before with tetra cycline (O.D.$_{650}$ = 0.6). The cultures were centrifuged (10 min, 5000 rpm) and suspended in 0.2 ml sucrose (25% in 50 mM Tris-HCl (pH 8)), 0.03 ml lysozyme (5 mg/ml) and allowed to stand for 5 min in ice. Twenty-five mM EDTA (0.08 ml) were added and the mixture again was allowed to stand for 5 min in ice. The cold mixture was then combined with 0.3 ml Triton X100 (2% in 50 mM Tris-HCl (pH 8), 60 mM EDTA) and allowed to stand in ice for 10 min. The lysed cells were centrifuged for 1 h at 17000 rpm and the resulting precipitate was dissolved in TES buffer (1 ml). The TES solution was supplemented with 0.1 vol 10 mg/ml ethidium bromide (Serva) and CsCl to 1 g/ml and centrifuged (35000 rpm, 48 h, 15°C). Two DNA bands could be visualized in the tube under UV-illumination. The band of highest density corresponds to plasmid form I DNA; the second band corresponds to form II and form III plasmid DNAs and some chromosomal DNA. The first band was collected from the tube, ethidium bromide removed by six isoamyl alcohol extractions, and the aqueous phase diluted with 3 vol water-supplemented with 0.2 M sodium acetate (pH 5.1) before DNA precipitation with 2.5 vol ethanol. The DNA was redissolved, extracted with phenol and again precipitated with ethanol. The form I DNA was digested to completion with PstI to remove the FMDV insert and the insert sized, as before, together with the uncleaved form I DNA on a 1% agarose gel using standard markers. Those clones containing inserts of ~750 bp were chosen for further analysis. The clones chosen were identified as follows:

*E. coli* HB101 (pBR322(Pst)/FMDV-703)
*E. coli* HB101 (pBR322(Pst)/FMDV-715)
*E. coli* HB101 (pBR322(Pst)/FMDV-410)
*E. coli* HB101 (pBR322(Pst)/FMDV-144)
*E. coli* HB101 (pBR322(Pst)/FMDV-331)
*E. coli* HB101 (pBR322(Pst)/FMDV-217)
*E. coli* HB101 (pBR322(Pst)/FMDV-118)
*E. coli* HB101 (pBR322(Pst)/FMDV-849)

This nomenclature indicates that the clone comprised an *E. coli* HB101 host and that the recombinant DNA molecule used to transform that host is plasmid pBR322, containing at the *Pst*I site an FMDV cDNA insert ("FMDV"), the particular clone being numbered arbitrarily from its position in the library of clones. *E.g.*, "401" denotes group 4, clone 1. For ease of identification, these clones shall hereinafter be referred to in shorthand form. For example, *E. coli* HB101 (pBR322(Pst)/FMDV-703) is identified as *E. coli* FMDV-703, its recombinant DNA molecule pFMDV-703 and the insert in that molecule or the polypeptide coded for by that insert FMDV-703.

Surprisingly none of the clones contained inserts greater than about 2500 bp in length. Because the size of the complete FMDV genome predicted from the FMDV RNA is about 7700 base pairs and reverse transcription of RNA to produce cDNA begins at the 3' end of the RNA, it was expected that none of these clones would code for proteins having the specificity of FMDV coat proteins VP1-VP4.

For further analysis 100 ml cultures of these clones were grown and the form I DNA isolated from them as above. Restriction maps of the *Pst*I cleaved DNA were prepared using *Eco*RI, *Bam*HI and *Hind*III, the size of the resulting fragments being determined as before. By comparing these restriction patterns and sizes with the restriction pattern of the cDNA determined previously, the location of each insert in the FMDV genome could be ascertained (Fig. 3). From this alignment and the inexact locations of the coding sequences for the viral proteins of FMDV (Fig. 1), the antigenic polypeptides which might be coded for by the DNA sequences of each insert could be predicted (Fig. 3). Surprisingly, the inserts of the chosen clones originated from various parts of the FMDV genome and did not all begin at the 3' end of that genome as would be the expected transcription pattern from AMV reverse transcriptase. Moreover, some of these inserts appeared to include a DNA sequence predicted to code for a portion of the capsid proteins of FMDV. *E.g.*, FMDV-715 and FMDV-144.

## SYNTHESIS OF A POLYPEPTIDE DISPLAYING THE SPECIFICITY OF FMD VIRAL ANTIGENS BY *E. COLI* CONTAINING RECOMBINANT DNA MOLECULES OF THIS INVENTION

Twenty-five ml cultures of *E. coli* FMDV-703, *E. coli* FMDV-715, *E. coli* FMDV-144, *E. coli* FMDV-331, and as negative controls *E. coli* HB101 and *E. coli* pBR322 were grown as before to stationary phase.* Chloroform was added to each culture and it was shaken for 10 min and the aqueous phase centrifuged. The pellet was redissolved in 1 ml buffer (50 mM Tris-HCl (pH 8) or 25 mM phosphate buffer with sodium chloride (pH 7)) and sonicated (Branson B-15) in ice for 3 min. After centrifugation, the pellet was resuspended in the previous buffer (1.0 ml) and again sonicated and centrifuged. The supernatants were combined and assayed for the presence of antigenic polypeptides reacting to antibodies of FMDV in several immuno assays.

---

* The assay results of these clones are reported in this example because the inserts of these clones together extend over a large portion of the FMDV genome.

1. Sandwich--Inhibition Assay

The wells of microtiter plates were coated with FMDV antibodies (hyperimmune antibodies, guinea pigs). The coated plates were then treated sequentially with the respective extracts prepared above, FMDV and FMDV hyperimmune antibodies covalently linked to alkaline phosphatase; a wash following each binding step. The color of the plates after further washing and addition of a solution of p-nitrophenylphosphate was monitored to determine any binding of the polypeptides in the bacterial extracts to anti-FMDV antibodies. Any FMDV antigenic polypeptides present in the bacterial extract will bind to the antibodies of the support. FMDV will only bind to the antibodies of the support where they have not been blocked with the antigenic polypeptides from the bacterial extract. Antibodies covalently linked to alkaline phosphatase will bind in far greater amount to virus than to any virus specific polypeptides of the extract, because the virus has many more antigenic determinants than the antigenic polypeptide. The reaction of p-nitrophenylphosphate with alkaline phosphatase produces a color. Therefore, a decrease in color from the control indicates that the extract contains polypeptides with the specificity of FMDV viral antigens. The results of this assay were as follows:

| Extract | Assay |
|---|---|
| E. coli FMDV-703 | - |
| E. coli FMDV-715 | + |
| E. coli FMDV-144 | + |
| E. coli FMDV-331* | + |
| E. coli pBR322 | - (negative control) |
| E. coli HB101 | - (negative control) |
| FMDV | +++ (positive control) |

* Infra.

2. Indirect Inhibition Assay

Two indirect inhibition assays were performed.

(a) Hyperimmune Antibody

Wells of microtiter plates were coated with FMD virus and the plates then treated with a reaction product of the respective bacterial extract and FMDV antibodies (hyperimmune antibody) (37°C for 30 min). The wells were washed several times and treated with FMDV anti-antibody convalently linked to alkaline phosphatase and again washed several times. As before, the color of the solid phase after treatment with a solution of p-nitrophenylphosphate was indicative of any immune reaction. In this assay, only those antibodies not bound by polypeptides containing FMDV specific sequences present in the bacterial extract will bind to FMDV on the solid phase. These bound sites will then provide the only sites available for anti-antibody binding. Therefore, a reduction in color indicates the presence of FMDV specific antigenic polypeptides in the bacterial extracts.

(b) Anti VPI Specific Antibody

This assay was performed as described above except that anti-VP1 specific antibody (mouse) was substituted for the hyperimmune antibody.

The results of the indirect inbibition assays were as follows:

| Extract | Assay (a) | Assay (b) |
|---|---|---|
| *E. coli* FMDV-703 | - | - |
| *E. coli* FMDV-715 | + | + |
| *E. coli* FMDV-144 | ±* | ±* |
| *E. coli* FMDV 331 | + | + |
| *E. coli* HB101 | - | - (negative control) |
| *E. coli* HB101 (pBR322) | - | - (negative control) |
| FMDV | - | not done (positive control) |

3. Direct Binding Assay - Anti VP1 Antibodies

In a procedure similar to that of the indirect inbibition assay, wells of microtiter plates were coated with bacterial extract from the respective clones. After washing, the wells were treated with anti VP1 specific antibody (mouse) covalently linked to alkaline phosphatase and the plates again washed. Again, the immune reaction was monitored by treatment of the solid phase with a solution of p-nitrophenylphosphate. In this assay, only those VP1 specific antibodies which bind to the FMDV specific polypeptides in the bacterial extract will be available for color reaction. Therefore, in this assay an increase in color indicates the presence of FMDV specific polypeptides in the extract. The results of this assay were as follows:

* "±" means some assays were weakly positive and others negative.

| Extract | Assay |
|---|---|
| _E. coli_ FMDV-703* | + |
| _E. coli_ FMDV-715 | ± |
| _E. coli_ FMDV-715** | + |
| _E. coli_ FMDV-144 | + |
| _E. coli_ FMDV-331 | ± |
| _E. coli_ HB101 | - (negative control) |
| _E. coli_ HB101 (pBR322) | - (negative control) |
| VP1 | +++ (positive control) |
| VP1 in HB101 extract | +++ (positive control) |

4. Radioimmunassay

Two radioimmunassays were performed as follows:

(a) Antibody Coated Discs

Polyvinyl discs were coated with FMDV antibody (hyperimmune antibody) substantially as described by S. Broome and W. Gilbert, "Immunological Screening Method To Detect Specific Translation Products", _Proc. Natl. Acad. Sci. USA_, 74, 75, pp. 2746-49 (1978). The solid phase was treated sequentially with the respective bacterial extract and radioactively-labelled hyperimmune antibody. Washing was carried out substantially as described by S. Broome and W. Gilbert, _supra_, except that 0.5% Nonidet P4 (Fluka) was added to the wash buffer for washing after binding of radioactive antibodies. The radioactivity of the solid phase was then monitored to determine the immune reaction. Here, radioactively labelled antibody will only bind to sites where an antigenic polypeptide from the bacterial extract has been bound to the antibody of the solid phase. Therefore,

---

* _Infra_.

** In this clone the DNA insert was reversed in orientation as compared to the usual pFMDV-715.

labelled solid phase indicates the presence of FMDV viral antigens in the bacterial extract.

(b) Bacterial Extract Coated Discs - Hyperimmune Antibody

Plastic discs (Polyvinyl film) were pre-soaked overnight in 0.2M $NaHCO_3$ (pH 9.2) and washed with water before use. Drops (~5 µl) of diluted bacterial extract (1:1 with 0.4 M $NaHCO_3$) were applied to the dry uncoated discs. Each disc was then placed in a single petri dish containing wet filter paper. The dish was closed and incubated 4 h at 37°C. The disc was then removed and washed three times (~25 ml) with phosphate buffer solution containing 0.1% BSA and 0.5% FCS and allowed to dry. A phosphate buffer solution (~1.5 ml) containing 0.1% BSA, 0.5% FCS, 0.05% Nonidet P40 ("WB/NP-40") and 5 x $10^6$ cpm $^{125}$I-IgG was applied to the disc by setting the disc on a nylon mesh which had been pre-soaked in wash buffer and incubating it overnight at 4°C in the buffer solution. After incubation, the disc was washed twice each with 25 ml WB/NP-40, phosphate buffer solution containing 0.5% Nonidet P40 and water. The discs were then dried and placed on a film for detection of radioactivity. Again, labelled solid phase indicates the presence of FMDV viral antigens in the bacterial extract.

The results of the radioimmunoassays were as follows:*

---

* As a further control the extracts of E. coli FMDV-703, E. coli FMDV-715, and E. coli FMDV-144 were assayed after the radioactive antibody had been incubated with virus. In each of these assays, the solid phase displayed no radioactivity demonstrating that all of the labelled antibody had been previously bound to the virus and was therefore not available for binding with the FMDV specific polypeptides of the bacterial extracts.

| Extract | Assay (a) | Assay (b) |
|---|---|---|
| *E. coli* FMDV-703 | + | + |
| *E. coli* FMDV-715 | + | + |
| *E. coli* FMDV-144 | + | + |
| *E. coli* FMDV-331 | ± | + |
| *E. coli* HB101 (pBR322) | - | - (negative control) |
| *E. coli* HB101 | - | - (negative control) |
| FMDV | +++ | +++ (positive control) |

## DNA FRAGMENT MAPPING AND NUCLEOTIDE SEQUENCE DETERMINATION

Apart from their use to produce polypeptides displaying FMDV antigenicity, the recombinant DNA molecules of this invention are also useful in replication of viral specific nucleotide sequences containing all or a portion of the genome of FMDV. FMDV DNA prepared in this way may be used to determine the nucleotide sequence of portions of the genome, particularly those portions coding for the various viral and capsid proteins of FMDV. From those sequences, the structure of the polypeptides themselves may be determined. Knowledge of these sequences not only aids in the study and understanding of the biology of FMDV but it permits modifications to be made in the recombinant DNA molecules of this invention to improve the yield of protein produced, to increase the activity of the proteins produced or to expand the virus serotype spectrum for which the proteins are effective.

Two of the clones, *E. coli* FMDV-715 and FMDV-144, whose extracts displayed positive assays for antigenic polypeptides of FMDV, were used to determine the nucleotide sequence of the region of the genome over which they extend (Figures 4 and 5). Based on the predicted location of these clones in the genome, this region includes that region which codes for at least a portion of capsid protein VP1.

The physical maps of the two inserts were constructed by digestion with various restriction enzymes (New England Biolabs or BRL) in the recommended buffers by well-known procedures. The products of digestion were electrophoresed on 1% agarose gels in 40 mM Tris-HOAc (pH 7.8), 2 mM EDTA. They were analyzed after visualization by staining with ethidium bromide and compared with the detailed physical map of pBR322 (J.G. Sutcliffe, supra). Restriction maps of the different plasmids were constructed on the basis of these digestion patterns. The maps were refined by sequencing the DNA inserts, substantially as described by A.M. Maxam and W. Gilbert, "A New Method For Sequencing DNA", Proc. Natl. Acad. Sci. USA, 74, pp. 560-64 (1977).

Plasmid DNA was prepared from the two clones as described previously and restricted by various restriction enzymes. Restricted DNA was dephosphorylated for 30 min at 65°C in the presence of calf intestinal phosphatase (Boehringer). Following phenol extraction and gel filtration on Sephadex G-75, the DNA was 5'-terminally labelled with γ-$^{32}$P-ATP (~6000 Ci/mmole) and polynucleotide kinase.

For sequencing, labelled fragments were handled in two ways. Some were purified on a polyacrylamide gel prior to cleavage with a second restriction enzyme. Others were immediately cleaved with a second restriction enzyme. In either case, the desired fragments were separated on a polyacrylamide gel in Tris-borate-EDTA buffer. Figure 4 displays the various restriction fragments (the circles indicating the label and the arrow the direction of sequencing) and the sequencing strategy employed using pFMDV-715 and pFMDV-144.

Most stretches of cDNA insert were sequenced from both strands and most restriction sites which served as labelled terminus were sequenced using a fragment spanning them. The relevant segment of the nucleotide sequence thus obtained for the insert of pFMDV-144 is depicted in Figure 5. This sequence does not exclude the possibility that modifications to the sequence such as mutations, including single or multiple, base substitutions, deletions, insertions, or inversions have not already occurred may not be employed subsequently to modify its expression, activity, or effective spectrum.

By comparing the polypeptide coded by this region of the FMDV genome with the sequence of 40 amino-terminal amino acids of authentic VP1, determined by K. Strohmaier et al., supra, it appears that the chosen reading frame is correct and that the DNA sequence of pFMDV-144 codes for about 90% of the capsid protein VP1 of FMDV, type O. After its poly G sequence, the DNA sequence of pFMDV-144 codes for a polypeptide whose amino acid sequence corresponds to amino acids 23-40 of that previously determined for VP1. The only differences between the previously determined amino acid sequence and that coded for by the DNA insert of pFMDV-144 are in amino acids 33 and 38, where the previous determinations were not definite, and in amino acid 37 where an Asn has been substituted for the previous Asp, a difference accountable by a single nucleotide change.* Therefore, the DNA insert pFMDV-144 is missing only amino acids 1-22 from its amino terminal end. The remainder of VP1, i.e., through its carboxy-terminal end, is coded for by the DNA insert of pFMDV-144 ("FMDV-144") because the insert extends much further than is necessary to code for the 240-250 amino acids of VP1.

The DNA insert of pFMDV-715 begins at amino acid 47 of VP1. It overlaps thereafter with the DNA

---

* In later isolated DNA fragment FMDV-1034, the nucleotide sequence coding for amino acid 37 was GAC, i.e., a codon coding for Asp (infra).

insert of pFMDV-144. *E.g.*, Fig. 3. Therefore, the insert of pFMDV-715 codes for about 80% of the capsid protein of VPI of FMDV, type O.

While neither of the inserts of pFMDV-144 or pFMDV-715 code for the complete amino acid sequence of VPI, both display activity consistent with viral specific polypeptides of FMDV in various assays. Therefore, each includes enough of a virus specific nucleotide sequence to produce an antigenic polypeptide in a host capable of expressing the recombinant DNA molecules of this invention (*supra*).

## IDENTIFICATION OF OTHER CLONES CONTAINING DNA INSERTS HYBRIDIZING TO THE DNA INSERTS OF THE RECOMBINANT DNA MOLECULES OF THIS INVENTION

Although the DNA inserts of *E. coli* FMDV-715 and *E. coli* FMDV-144, do not include the complete sequence for a particular viral protein, each produces bacterial extracts which display the antigenicity of viral polypeptides of FMDV. These clones may therefore be employed to select other clones which may contain more complete or additional DNA sequences and which in a host may be capable of expressing antigenic polypeptides. Such screening of clones was carried out by isolating the form I DNA of the particular clone and employing its DNA insert to hybridize the form I DNA from other clones in similar manner to that used to hybridize the FMDV RNA in the original clone screening process (*supra*). For example, the insert of pFMDV-144 has been employed to locate other clones containing DNA inserts hybridizing to a portion of the insert of pFMDV-144.

Form I DNA from pFMDV-144 was isolated as before and the *Pst*I - *Hind*II fragment (closest to the 5' end) isolated. This fragment contains about 90% of the nucleotide sequence coding for VPl. A total of 1000 clones (400 from the previous library and 600 (12 groups of 50 clones) from a second transformation) were screened using the above fragment as a probe in a similar manner to the screening of the original library with the FMDV-RNA probe. Sixteen clones displayed strong hybridization to this fragment. They were further analyzed by Bam HI restriction mapping and sizing. Three clones having DNA inserts with Bam HI restriction sites and of more than about 1000 bp in length were selected (Figure 3). These clones were identified as before by their arbitrary position in the library of clones -- *E. coli* HB101 (pBR322(Pst)/FMDV-1034), *E. coli* HB101 (pBR322(Pst)/FMDV-1824) and *E. coli* HB101 (pBR322(Pst)/FMDV-1933). Because of their strong hybridization to FMDV-144, the presence of the Bam HI restriction site and the size of their Bam HI restriction fragments, these clones are expected to include the complete nucleotide sequence of VP1 and perhaps all or a portion of the nucleotide sequences of VP2 and VP3. In any event additional clones from a library may be screened according to the processes of this invention to select such clones.

Cultures of the three clones were prepared as before, and their bacterial extracts assayed by the direct binding assay-anti VPI antibodies ("3") and the bacterial extract coated discs-hyperimmune antibody radioimmune assay ("4(b)") described previously. The results of these assays were as follows:

| Extract | Assay 3 | Assay 4(b) |
|---|---|---|
| *E. coli* FMDV-1034 | + | + |
| *E. coli* FMDV-1824 | + | + |
| *E. coli* FMDV-1933 | + | + |
| *E. coli* HB101 (pBR322) | - | - (negative control) |
| *E. coli* HB101 | - | not done (negative control) |

| Extract | Assay 3 | Assay 4(b) |
|---|---|---|
| FMDV | not done | +++ (positive control) |
| VPI | +++ | not done (positive control) |
| VPI in HB101 extract | +++ | not done (positive control) |

This screening process may, of course, be employed to screen additional clones using the FMDV-144 fragment to detect other clones having inserts hybridizing to this fragment or it may be employed using other fragments to detect clones having inserts hybridizing to them.

CLONES CONTAINING pFMDV-703 AND pFMDV-331

From their location in the FMDV genome, the DNA inserts of pFMDV-703 and pFMDV-331 do not include even a portion of the nucleotide sequence of VP1. Nor do they hybridize to FMDV-144 (supra). Therefore, the antigenic polypeptides produced by pFMDV-703 and pFMDV-331 in a host capable of expressing polypeptides displaying the specificity of viral antigens of FMDV are not expected to be identical to VP1. However, bacterial extracts of cultures of FMDV-703 and FMDV-331 clones display antigenic activity against hyperimmune antibody and VP1 specific antibody in some assays. It is believed that this activity indicates that the antigenic polypeptides expressed by E. coli FMDV-703 and E. coli FMDV-331 contain amino acid sequences which act as antigenic determinants for VP1 antibodies. Therefore, these antigenic polypeptides may be employed in methods and compositions for rendering animals resistant to FMDV for at least a time.

IMPROVING THE YIELD AND ACTIVITY OF POLYPEPTIDES DISPLAYING THE SPECIFICITY OF VIRAL ANTIGENS OF FMDV PRODUCED IN ACCORDANCE WITH THIS INVENTION

The level of production of a protein is governed by two major factors: the number of copies of its gene within the cell and the efficiency with which those gene copies are transcribed and translated. Efficiency of transcription and translation (which together comprise expression) is in turn dependent upon nucleotide sequences, normally situated ahead of the desired coding sequence. These nucleotide sequences or expression control sequences define, *inter alia*, the location at which RNA polymerase interacts to initiate transcription (the promoter sequence) and at which ribosomes bind and interact with the mRNA (the product of transcription) to initiate translation. Not all such expression control sequences function with equal efficiency. It is thus of advantage to separate the specific coding sequences for the desired protein from their adjacent nucleotide sequences and fuse them instead to other known expression control sequences so as to favor higher levels of expression. This having been achieved, the newly engineered DNA fragment may be inserted into a multicopy plasmid or a bacteriophage derivative in order to increase the number of gene copies within the cell and thereby further improve the yield of expressed protein.

Several expression control sequences may be employed as described above. These include the operator, promoter and ribosome binding and interaction sequences (including sequences such as the Shine-Dalgarno sequences) of the lactose operon of *E. coli* ("the *lac* system"), the corresponding sequences of the tryptophan synthetase system of *E. coli* ("the *trp* system"), the major operator and promoter regions of phage λ ($O_LP_L$ and $O_RP_R^I$), the control region of *Filamenteous* single stranded DNA phages, or other sequences which control the expression of genes of prokaryotic or eukaryotic cells and their viruses. Therefore, to improve the production of a particular polypeptide in an appropriate host, the gene coding for that polypeptide may be selected as before and removed from a recombinant DNA molecule containing it and reinserted into a recombinant DNA molecule closer or in a more appropriate relationship* to its former expression control sequence or under the control of one of the above improved expression control sequences. Such methods are known in the art.

Further increases in the cellular yield of the desired products depend upon an increase in the number of genes that can be utilized in the cell. This is achieved, for illustration purposes, by insertion of recombinant DNA molecules engineered in the way described previously into the temperate bacteriophage λ (NM989), most simply by digestion of the plasmid with a restriction enzyme, to give a linear molecule which is then mixed with a restricted phage λ cloning vehicle (e.g., of the type descdribed by N. E. Murray et al., "Lambdoid Phages That Simplify The Recovery Of In Vitro Recombinants" Molec. gen. Genet. 150, pp. 53-61 (1977) and N. E. Murray et al., "Molecular Cloning Of The DNA Ligase Gene From Bacteriophage T4", J. Mol. Biol., 132, pp. 493-505 (1979)) and the recombinant DNA molecule recircularized by incubation with DNA ligase. The desired recombinant

---

* As used herein "relationship" may encompass many factors, e.g., the distance separating the expression enhancing and promoting regions of the recombinant DNA molecule and the inserted DNA sequence, the transcription and translation characteristics of the inserted DNA sequence or other sequences in the vector itself, the particular nucleotide sequence of the inserted DNA sequence and other sequences of the vector and the particular characteristics of the expression enhancing and promoting regions of the vector.

phage is then selected as before and used to lysogenise a host strain of E. coli.

Particularly useful λ cloning vehicles contain a temperature-sensitive mutation in the repression gene cI and suppressible mutations in gene S, the product of which is necessary for lysis of the host cell, and gene E, the product which is the major capsid protein of the virus. With this system the lysogenic cells are grown at 32°C and then heated to 45°C to induce excision of the prophage. Prolonged growth at 37°C leads to high levels of production of the protein, which is retained within the cells, since these are not lysed by phage gene products in the normal way, and since the phage gene insert is not encapsidated it remains available for further transcription. Artificial lysis of the cells then releases the desired product in high yield.

In addition, it should be understood that the yield of polypeptides displaying FMDV antigenicity prepared in accordance with this invention may also be improved by substituting different codons for some or all of the codons of the present DNA sequences, these substituted codons coding for amino acids identical to those coded for by the codons replaced.

Finally, the activity of the polypeptides produced by the recombinant DNA molecules of this invention may be improved by fragmenting, modifying or derivatizing the DNA sequences or polypeptides of this invention by well-known means, without departing from the scope of this invention.

## CLONES CONTAINING A PART OF THE pFMDV-1034 DNA INSERT

As one example of a process to improve the yield and therefore the activity of polypeptides displaying the specificity of viral antigens of FMDV produced in accordance with this invention, pFMDV-1034 (Figures 3 and 6) was isolated from colonies of E. coli HB 101 (pBR322(Pst)/ FMDV-1034). This hybrid plasmid was employed as the source of a DNA sequence for subsequent insertion into expression control vector pPLc24.

1. Vector pPLc24

Vector pPLc24 (Figure 6, a gift of Walter Fiers) contains the β-lactamase gene and part of pBR322. A 290 base pair HaeII-EcoRI fragment contains the $P_LO_L$ region from bacteriophage λ. The direction of transcription from the $P_L$ promoter is towards the EcoRI. A 431 base pair EcoRI-BamHI fragment codes for the ribosome binding site and the first 99 amino acid residues of the bacteriophage MS2 replicase gene, obtained from plasmid pMS-7 (R. Devos et al., "Construction And Characterization Of A Plasmid Containing A Nearly Full-Size DNA Copy Of Bacteriophage MS2 RNA", J. Mol. Biol. 128, 595-619 (1979)). Translation of the MS2 replicase protein fragment runs colinearly with the transcription from the $P_L$ promoter.

Transcription from the $P_L$ promoter -- present on plasmid pPLc24 -- is repressed by maintaining the plasmid in an E. coli strain that synthesizes a repressor protein, the product of the phage gene cI. Due to its autoregulating mode of synthesis (M. Ptashne et al., "Autoregulation And Function Of A Repressor In Bacteriophage λ", Science, 194, pp. 156-61 (1976)), one copy of the cI phage gene on the chromosome of a lysogenic strain is able to repress fully the $P_L$ promoter present on a multicopy plasmid.

2. Preparation and Selection of Clones Containing A Part Of The pFMDV-1034 DNA Insert

Referring now to Figure 7, vector pPLc24 was restricted with restriction enzymes BamHI and HindIII to give complementary ends for subsequent ligation with restricted DNA sequence from pFMDV-1034.

Cultures of E. coli HB 101 (pBR322(Pst)/FMDV-1034) were prepared at 37° in bactotryptone medium, supplemented with 10 μg/ml tetracycline ($O.D._{650}$ = 0.6). The cultures were centrifuged (10 min, 5000 rpm) and resuspended in 0.2 ml sucrose (25% in 50 mM Tris-HCl (pH 8)) and 0.03 ml lysozyme (5 mg/ml) and allowed to stand for 5 min in ice. Twenty-five mM EDTA (0.08 ml) were added and the mixture again was allowed to stand for 5 min in ice. The cold mixture was then combined with 0.3 ml Triton X100 (2% in 50 mM Tris-HCL (pH 8), 60 mM EDTA) and allowed to stand in ice for 10 min. The lysed cells were centrifuged for 1 h at 17000 rpm and the resulting supernatant was supplemented with 0.1 vol 10 mg/ml ethidium bromide (Serva) and CsCl to 1 g/ml and centrifuged (35000 rpm, 48 h, 15°C). Two DNA bands could be visualized in the tube under UV-illumination. The band of highest density corresponds to plasmid form I DNA (pFMDV-1034; Figure 6); the second band corresponds to form II and form III plasmid DNAs and some chromosomal DNA. The first band was collected from the tube, ethidium bromide removed by six isoamyl alcohol extractions, and the aqueous phase diluted with 1 vol water-supplemented with 0.2 M sodium acetate (pH 5.1) before DNA precipitation with 2.5 vol ethanol. The DNA was redissolved, extracted with phenol and again precipitated with ethanol. The form I DNA was digested to completion with PstI and a fragment (FMDV-1034) of 1150 base pairs, representing the total DNA insert of pFMDV-1034, was isolated on a 1% agarose gel using standard size markers (Figure 8). This insert was then further restricted with BamHI and HindIII using conventional restriction procedures. The BamHI-HindIII restriction results in a DNA fragment ("FMDV-1034 (BamHI-HindIII)") of about 850 base pairs, about 150 base pairs having been removed from each end of the PstI-pFMDV-1034 fragment by the restriction (Figures 7-10).

The cleaved vector (pPLc24 (BamHI-HindIII)) and DNA fragment (FMDV-1034 (BamHI-HindIII)) were combined in a ratio of 1:2 and precipitated with ethanol. The precipitate was separated and redissolved in T4 DNA ligase buffer (3 μg vector/ml) and ligation allowed to proceed overnight at 15°C.

$CaCl_2$ competent E. coli W6 ($\lambda_{rex}$), having chromosomal λ repressor cI so as to prevent $P_L$ controlled expression, was transformed with the ligase mixture and ampicillin resistant colonies selected after growth in L-broth supplemented with 40 μg/ml ampicillin. Because vector pPLc24 includes the gene coding for β-lactamase, E. coli hosts which have been transformed with a plasmid having that gene intact will grow in medium containing ampicillin to the exclusion of those bacteria not so transformed.

Fifty ampicillin-resistant clones were picked and hybridized, as described by M. Grunstein and D. S. Hogness, "Colony Hybridization: A Method For The Isolation of Cloned DNAs That Contain A Specific Gene", Proc. Natl. Acad. Sci. USA, 72, pp. 3961-65 (1975), with a $^{32}P$-labelled PstI-pFMDV-1034 probe prepared by nick translation in the presence of E. coli DNA pol I and $\alpha$-$^{32}P$-dATP. Seven intensely positive clones were selected. These seven clones may be identified as follows: E. coli W6 ($\lambda_{rex}$-pPLc24 (BamHI-HindIII)/FMDV-1034 (BamHI-HindIII). These clones will be referred to subsequently as E. coli W6 ($\lambda_{rex}$-pPL-VP1-1) their plasmids as pPL-VP1-1 and their DNA inserts as VP1-1, respectively*.

---

* A second clone identified as E. coli W6 ($\lambda_{rex}$-pPL-VP1-5) was also selected. In subsequent assays, colonies of E.coli NF1 ($\lambda N^-cro^-cI_{ts}$-pPL-VP1-5) produced bacterial extracts that displayed responses consistent with the presence of VP1-related antigens after growth overnight at 37°C.

Plasmid DNA was isolated from these intensely hybridizing clones as before and employed to transform ($CaCl_2$ competent) bacterial strain E. coli NF1 (K12M72 $lac_{am}$ $\Delta_{trp}$ EA2 $Sm^R$ (λcI851 $N_{am7}N_{am53}$ ΔHI $bio^-$) ("E. coli NF1 (λ$N^-$$cro^-$$cI_{ts}$)") (U. H. Bernard et al., "Construction Of Plasmid Cloning Vehicles That Promote Gene Expression From The Bacteriophage λ $P_L$ Promoter", Gene, 5, 59-76 (1979)). This strain harbors a defective, non-excisable λ prophage carrying a mutant cI gene. The mutant gene codes for a temperature-sensitive repressor ($cI_{ts}$), thus allowing turn on transcription from the $P_L$ promoter by shifting the temperature -- at 28°C the repressor is active and represses transcription, but at 42°C the repressor is inactivated and transcription from the $P_L$ promoter is fully turned on.

The ΔHI deletion of the prophage removes part of the cro gene and all other genes further to the right of cro (M. Castellazzi et al., "Isolation And Characterization Of Deletions In Bacteriophage λ Residing As Prophage In E. coli K12", Mol. gen. Genet., 117, pp. 211-18 (1972)). The deletion of the cro gene is advantageous because accumulation of the cro protein is known to repress transcription from the $P_L$ promoter (A. Johnson et al., "Mechanism Of Action Of The cro Protein Of Bacteriophage λ", Proc. Natl. Acad. Sci. U.S.A. 75, 1783-1787 (1978)). Strain NF1 has two amber mutations in N rendering it functionally N-negative. Therefore, in strain NF1 transcription proceeds in the absence of the N-gene product.

Other strains, e.g., E. coli M5219 (K12 M72 $lac_{am}$ $trp_{am}$ $Sm^R$ (λcI857 ΔHI bio252) (H. Grier, "The Kil Gene of Bacteriophage λ", Virology, 66, pp. 589-604 (1975)), which express the N-gene product could also be employed in this invention. These N+ strains could be advantageous in instances where the DNA regions to be transcribed contain transcription terminator-like sequences or slow-down sequences for the RNA polymerase. For example, the product of the *N* gene is known to act as an anti-terminator in bacteriophage λ (J. W. Roberts, "Transcription Termination And Late Control In Phage λ", *Proc. Natl. Acad. Sci. U.S.A.*, 72, pp. 3300-04 (1975)). The anti-termination effect was equally observed with terminator sequences not naturally present on phage λ DNA (e.g., the natural stop at the end of the *trp* operon), provided the RNA transcript starts at the $P_L$ promoter. Furthermore, polarity effects, introduced by the presence of a nonsense codon in the $P_L$ transcript, were relieved under the action of the *N*-gene protein (for review see N. Franklin and C. Yanofsky, "The *N* Protein Of λ: Evidence Bearing On Transcription Termination, Polarity And The Alteration Of *E. coli* RNA Polymerase" in *RNA Polymerase* (Cold Spring Harbor Laboratory, 1976) pp. 693-706).

Having the aforementioned plasmid in a thermo-inducible bacterial *cI* background allows experimental switching on or off of the activity of $P_L$ promoter.

Colonies of the transformed hosts were grown in L-broth, supplemented with 40 μg/ml ampicillin, at 28°C and clones selected at random from each of the seven sets of transformants. In addition, colonies of hosts transformed with plasmid pPLc24, were grown at 28°C and clones selected at random from these transformants. These clones were identified as follows:

*E. coli* NF1 ($\lambda N^-cro^-cI_{ts}$-pPL-VP1-1)
*E. coli* NF1 ($\lambda N^-cro^-cI_{ts}$-pPLc24)

3. Radioimmunassay of Bacterial Extracts From *E. coli* NF1 ($\lambda N^-cro^-cI_{ts}$-pPL-VP1-1)

Radioimmunassays of bacterial extracts from *E. coli* NF1 ($\lambda N^-cro^-cI_{ts}$-pPL-VP1-1) were performed as follows:

(a) Anti-$O_1$K Coated Discs -- Labelled Anti-VP1 Antibody

Two clones of E. coli NF1 ($\lambda N^- cro^- cI_{ts}$-pPL-VP1-1) were randomly selected from the NF1 colonies described above. These were grown at 28°C and 42°C as before, in duplicate, and tested for expression of antigenic polypeptides of FMDV by Broome-Gilbert assay using anti-$O_1$K* coated discs (S. Broome and W. Gilbert, "Immunological Screening Method To Detect Specific Translation Products", Proc. Natl. Acad. Sci. USA, 75, pp. 2746-49 (1978)).

The extracts were prepared from 100 ml cultures grown at 28°C to a density of $6x10^8$ cells/ml at which point the temperature was raised to 42°C and incubation continued for 4 h. The cells were then centrifuged at 6000 rpm for 10 min and the pellet redissolved in 1 ml buffer (50 mM Tris-HCl (pH 8) or 25 mM phosphate buffer with sodium chloride (pH 7)) and sonicated (Branson B-15) in ice for 1 min. After centrifugation, the pellet was resuspended in the previous buffer (0.1 ml) and again sonicated and centrifuged. The supernatants were combined and assayed in duplicate as described below.

Polyvinyl discs were coated, substantially as described by Broome and Gilbert, supra, with anti-$O_1$K IgG from guinea pig. The solid phase was treated sequentially with the 5 µl of the respective bacterial extracts and $^{125}$I-labelled IgG anti-VP1 from mouse that had previously been exhausted with a bacterial extract from E. coli HB 101 (pBR322)** so that improved negative

---

* Guinea pig hyperimmune serum to FMDV, Type O, Subtype 1 (Kaufbeuren).

** The $^{125}$I-labelled IgG anti-VP1 was incubated at 4°C for 4-6 h with 0.5 ml of the extract ($\cong 5x10^9$ bacteria) and centrifuged to remove any immune complexes.

controls might be had. Washing was carried out substantially as described by Broome and Gilbert, supra, except that 0.5% Nonidet P4 (Fluka) was added to the wash after binding of the radioactive antibodies. Two discs were used for each assay.

In this assay, radioactively labelled anti-VP1 will only bind to sites on the disc where a VP1 related antigen from the bacterial extract has been previously bound to the hyperimmune antibody of the solid phase. Therefore, labelled solid phase indicates the presence of VP1 related antigens in the extract. The results were as follows:

| Extract | Assay Disc 1 #1 | Assay Disc 1 #2 | Assay Disc 2 #3 | Assay Disc 2 #4 |
|---|---|---|---|---|
| NF1 ($\lambda N^{-}cro^{-}cI_{ts}$-pPL-VP1-1) | +++ | +++ | +++ | +++ |
| NF1 ($\lambda N^{-}cro^{-}cI_{ts}$-pPLc24) | - | - | - | - |
| $O_1K$ (positive control)* | +++ | +++ | +++ | +++ |
| pBR322 (negative control) | - | - | - | - |

In addition, the above described bacterial extracts of *E. coli* NF1 ($\lambda N^{-}cro^{-}cI_{ts}$-pPL-VP1-1) displayed positive results in the Broome-Gilbert assay at up to 1:50 dilution. This compares favorably with the positive assay observed for a 1:250 dilution of the positive control FMDV $O_1K$.

(b) Anti-$O_1K$ Coated Discs -- Virus Exhausted Labelled Anti-VP1 Antibody

Using the above prepared bacterial extracts, a similar assay was conducted except that prior to

---

* FMDV, Type O, Subtype 1 (Kaufbeuren), 5 μl (0.4 mg/ml).

treatment of the solid phase bound antigens from the bacterial extract with $^{125}$I-labelled IgG anti-VP1, the labelled antibody was exhausted as before with E. coli HB 101 (pBR322) and then exhausted with FMDV, type $O_1K$. In this assay, the former positive results for E. coli NF1 ($\lambda N^{-}cro^{-}cI_{ts}$-pPL-VP1-1) bacterial extract and the positive control were negative because all of the labelled anti-VP1 had been exhausted with virus and therefore was not available for binding to any VP1 antigenic determinants bound to the antibodies of the solid phase. These results demonstrate clearly that FMDV $O_1K$ completely binds VP1 specific antibody and that the antigens of the bacterial extract do not bind to that combination.

### (c) Anti-$O_1K$ Coated Discs -- Labelled FMDV and Bacterial Extract Competition

In this assay anti-$O_1K$ coated discs were prepared as before and employed in a competition between $^{125}$I-labelled FMDV, type $O_1K$, and bacterial extract from E. coli NF1 ($\lambda N^{-}cro^{-}cI_{ts}$-pPL-VP1-1) prepared previously. In this assay, the extract, if it contains any VP1 antigenic determinants, and labelled virus compete directly for the binding sites on the anti-$O_1K$ bound solid phase. The results of the assay were as follows:

| Extract | Assay |
|---|---|
| NF1($\lambda N^{-}cro^{-}cI_{ts}$-pPL-VP1-1)/$^{125}$I-FMDV $O_1K$ | + |
| NF1($\lambda N^{-}cro^{-}cI_{ts}$-pPLc24)/$^{125}$I-FMVD $O_1K$ | +++ |
| $^{125}$I-FMDV $O_1K$ (positive control) | +++ |

Therefore, the bacterial extract of E. coli NF1 ($\lambda N^{-}cro^{-}cI_{ts}$-pPL-VP1-1) competes successfully with labelled virus for binding to the anti-$O_1K$ coated discs. This competition is not observed with the bacterial extract of E. coli NF1 ($\lambda N^{-}cro^{-}cI_{ts}$-pPLc24).

The observed competition is further evidence of the presence of VP1 antigenic polypeptides in the bacterial extracts of E. coli NF1 ($\lambda N^{-}cro^{-}cI_{ts}$-pPL-VP1-1) and demonstrates that those antigens are able to complete successfully with the authentic VP1 antigens of the FMDV virus.

(d) Anti-$O_1$K Coated Discs -- Labelled IgG Against Freund's Adjuvant

Using a procedure substantially identical to (a) above, anti-$O_1$K coated discs were treated sequentially with the above described bacterial extracts and $^{125}$I-labelled IgG against Freund's adjuvant. No positive results were observed. These results demonstrate that the binding of labelled antibody to the antigens of the bacterial extract is specific to VP1 antibodies.

4. Expression of pPL-VP1-1 In Minicells

E. coli DS 410 (J. N. Reeve, Mol. gen. Genetics, 158, pp. 73-79 (1977)) was transformed with form I DNA isolated as before from E. coli W6 ($\lambda_{rex}$-pPL-VP1-1) and E. coli W6 ($\lambda_{rex}$-pPLc24). These transformations were carried out under substantially identical conditions to those described previously except that the transformations were carried out in the presence of pRK248 $cI_{ts}$ (a gift of Walter Fiers) (H. Bernard and D. R. Helinski, Methods in Enzymology, in press (1980)), in order to introduce a temperature sensitive λ repressor ($cI_{ts}$) into the transformed strains.

Colonies of the transformants were grown on L-plates (28°C), supplemented with tetracycline (10 μg/ml) and ampicillin (40 μg/ml), to select those colonies having doubly transformed clones -- i.e. containing pPLc24 or pPL-VP1-1 and pRK248. Since pPLc24 carries the gene for ampicillin resistance and pRK248 carries the gene for tetracycline resistance, growth in the above ampicillin and tetracycline supplemented medium distinguishes doubly transformed clones from those not so transformed.

Several of these doubly transformed clones were picked onto L-plates, supplemented as before, and colonies again grown at 28°C. To ensure that the pPL-VP1-1 transformed clones from the second incubation still contained the VP1-1 DNA insert, a number of these clones were picked and plasmid form I DNA separated therefrom as described previously. In all cases, the form I DNA separated from these clones had the correct size for pPLc24 with inserted VP1-1 DNA.

One clone of E. coli DS 410 (pPL-VP1-1)/(pRK248) and one clone of E. coli DS 410 (pPLc24)/(pRK248), were selected at random from the several incubations described above and used to produce minicells. (J.N. Reeve, "Mucopeptide Biosynthesis By Minicells In Escherichia coli", J. Bacteriol., 131, pp. 363-65 (1977); J.N. Reeve, Mol. gen. Genetics, 158, pp. 73-79 (1977)). Cells were grown in L-broth at 28°C and labelled during incubations at 42°C for 30 min with $^{35}S$-Methionine.

The labelled products were directly analyzed by separation on a 12.5% SDS-polyacrylamide gel and subsequent autoradiography of the dried gel. A strong protein band was observed in cells carrying the plasmid pPL-VP1-1 but not in cells carrying plasmid pPLc24. The molecular weight of the observed protein (≅45000-48000d.) is in good agreement with that of a fusion protein consisting of the 99 amino acids of MS2, the amino acids coded for by the about 850 base pair VP1-1 insert and that portion of pBR322 (13 amino acids) that precedes the first stop codon (Figures 7 and 9-10).

## 5. Antibody Precipitation Of The Polypeptides Synthesized In Minicells

The above described $^{35}S$-Methionine labelled cells were treated with lysozyme/NP-40 and the cell extracts pre-adsorbed with Protein A Sepharose (Pharmacia) and centrifuged in an Eppendorf tube for 2 min. This procedure results in the extracts being unspecifically bound to the Protein A Sepharose.

The supernatant from these tubes was incubated for 30 min at 37°C with purified IgG from mouse anti-VP1 in order to bind the VP1 related antigens of the extracts. Protein A Sepharose was added to bind the antibody-antigen complex and the mixture incubated at 4°C for 2 h. The Protein A Sepharose was washed four times with TNE(10 mM Tris-HCl(pH 7.4), 100 mM NaCl, 1mM EDTA)/0.05% NP-40 and the retained products were separated on a 12.5% SDS-polyacrylamide gel.

The protein bands were observed by autoradiography. A strong band at ≅45000-48000d and a weaker band at ≅25000d (perhaps processed VP1) were observed from the antibody bound extracts from pPL-VP1-1 transformed cells. Neither of these bands was detected in the antibody bound extracts from pPLc24 transformed cells. Therefore, the bacterial extracts of E. coli DS 410 (pPL-VP1-1)/(pRK248) contain polypeptides which may be precipitated with anti-VP1 antibodies, while the polypeptides contained in the bacterial extracts of E. coli DS 410 (pPLc24)/(pRK248) are not precipitated by anti-VP1 antibodies because they do not contain VP1 specific antigens.

## 6. Estimation Of Production Of Polypeptides Displaying FMDV Antigenicity

In order to estimate the production of proteins by these transformed hosts, the above described stronger protein band -- 45000-48000d --and the band corresponding to β-lactamase (27000d) were excised from the gel and each incubated with NCS solubilizer (Amersham) and scintillation liquid to release the protein from the gel. The radioactivity of the protein solutions was then determined in a scintillation counter.

Calculating that one molecule of the fused polypeptide (45000-48000d) contains 5 methionines (from sequence data set forth later and the known sequences of MS2 and pBR322) and that $10^9$ minicells have been applied to the gel (most not containing plasmids), one cell would produce 2 molecules of fused protein and 0.5 molecules of β-lactamase.

Experiments using the amplification method and "normal" cells (NF1) (Neidhardt et al., J. Bacteriol. July, 1980) permitted determination of a lower limit of about 100 molecules/cell/30 min labelling.

This estimated lower limit is consistent with protein production estimates -- ≅300 molecules/cell -- made on the bases of the extract dilutions discussed previously. Of course, it should be understood that these determinations are merely approximate and furthermore that the labelling time, the density of the culture, or even the chosen host may not be optimal for expression of a VP1-containing fusion protein.

## DNA FRAGMENT MAPPING AND NUCLEOTIDE SEQUENCE DETERMINATION

In order to determine the nucleotide sequence of the gene that codes for VP1 as well as the nucleotide sequence of the VP1-1 DNA insert and the amino acid sequences coded for by these genes, pFMDV-1034 was isolated from colonies as described previously and that plasmid used to determine the nucleotide sequences of the FMDV insert. This plasmid was chosen because it includes at least the full structural gene for VP1.

The physical map of this FMDV region was constructed, as described previously, by digestion with various restriction enzymes (New England Biolab or BRL) in the recommended buffers by well-known procedures. The products of digestion were electrophoresed on agarose gels in 40 mM Tris-HOAc (pH 7.8), 2 mM EDTA. They were analyzed after visualization by staining with ethidium bromide and compared with the detailed physical map of pBR322 (J. G. Sutcliffe, "Complete Nucleotide Sequence Of The *Escherichia coli* Plasmid pBR322", Cold Spring Harbor Symposium, 43, I, pp. 77-90 (1978)). A restriction map of the insert was constructed on the basis of these digestion patterns (Figure 8) and the map refined by sequencing the restricted DNA, substantially as described by A.M. Maxam and W. Gilbert, "A New Method For Sequencing DNA", *Proc. Natl. Acad. Sci. USA*, 74, pp. 560-64 (1977).

Figure 8 displays the various restriction fragments (the circles indicating the label and the arrow the direction of sequencing) and the sequencing strategy employed. Figures 9-10 display the relevant portion of the nucleotide sequence of the DNA insert FMDV-1034. By comparing the polypeptide coded for by this DNA insert with the sequence of 40 amino-terminal amino acids of authentic VP1, determined by K. Strohmaier et al. *supra*, it appears that the chosen reading frame is correct and that nucleotides 1-639 of FMDV-1034 code for all of the capsid protein VP1 of FMDV, type O, i.e. comprise the structural gene of VP1 (Figures 9-10).

Amino acids 1-40 FMDV-1034 correspond well with the previously determined VP1 amino acid sequence except at amino acids 33 and 38, where the previous determinations were questionable. The C-terminus of VP1 is uncertain. However, based on unpublished results of Strohmaier et al. reporting preliminary COOH-terminal amino acid sequence data and amino acid composition data for peptide fragments (20-30 amino acids) from the COOH-terminal end of VP1, the protein appears to extend to about amino acid 213 of the depicted sequence. Therefore, the remainder of the insert most likely consists of nucleotides of the FMDV genome other than that which code for VP1.

In addition, the nucleotide and amino acid sequences depicted in Figures 9-10 permit determination of the nucleotide sequence and amino acid sequence coded thereby of DNA insert VP1-1. This insert extends from the BamHI restriction site in FMDV-1034 -- amino acid 9 of VP1 -- through the HindIII restriction site in FMDV-1034. This latter cut is located about 79 amino acids after amino acid 213, the estimated COOH terminus of VP1. Therefore, the FMDV related insert in VP1-1 consists of about 852 base pairs coding for 284 amino acids.

The determined nucleotide sequence of VP1-1, the known sequence of MS2 and pBR322 and a determination of the nucleotide sequences at the fusion of VP1-1 to MS2 and pBR322 support the conclusion that the protein expressed by E. coli NF1 ($\lambda N^{-}cro^{-}cI_{ts}$-pPL-VP1-1) is a fusion product consisting of the 99 amino acids of MS2 present in pPLc24, the about 205 amino acids of VP1 (amino acids 9-213), the about 79 amino acids coded for by the additional nucleotides of the FMDV genome present in VP1-1 and the about 13 amino acids of pBR322 that precede the first stop codon in the correct reading frame. The size of this fusion protein is consistent with the ≅45000-48000d protein isolated in the minicells discussed previously.

It is important to note that although the insert in pPL-VP1-1 does not code for the complete amino acid sequence of VP1, it includes enough of the VP1 specific nucleotide sequence to produce a fusion product which displays the specificity of a viral antigen of FMD (supra).

In addition, it should be recognized that this invention is not limited to that particular fusion protein. Rather, the complete VP1 protein or other antigenically specific fragments or derivatives thereof including polypeptides that contain only a part of VP1 or polypeptides that include amino acids in addition to those of VP1 or fragments thereof, either alone or as part of fusion proteins, may be produced by methods analogous to those illustrated by way of example above and used in methods and compositions for protecting animals for at least some time from FMD viral infection. For example, pFMDV-1034 could be cleaved with PstI and HindIII and the respective ends chewed back, e.g., by exonuclease treatment, until the ends are close to amino acid 1 and 213 of VP1, e.g., to about amino acid -10 and 220 or amino acid 15 and 150, before preparing the fragment for insertion in the expression control vector. Likewise, the BamHI/HindIII fragment of pFMDV-1034 might be hybridized to an appropriate single stranded fragment of pFMVD-1034 or other DNA sequence containing the full VP1 structural gene and that full gene used as a template to extend the BamHI/HindIII fragment to the beginning, closer to the beginning or beyond the beginning of the VP1 coding sequence. Finally, other fragments or derivatives of the VP1 gene, whose polypeptides possess antigenic activity to FMDV could be prepared for use by the processes of this invention, e.g., by variation of the above described manipulations.

In one example of such manipulations pPL-VP1-1 was digested with BamHI using the conditions recommended by the supplier and 40 μg of the digested fragment mixed with three units of Bal 31 (BRC) in 750 μl of Bal 31 buffer (12 mM $CaCl_2$, 12 mM $MgCl_2$, 600 mM NaCl, 20 mM Tris-HCl (pH 8.1), 1 mM EDTA). Aliquots were taken after 2, 6, 10, 14 and 20 min of incubation and phenolized as usual to destroy any remaining Bal 31 and precipitated with EtOH. The resultant DNA fragments were recircularized by blunt-end ligation to form plasmids having various length deletions on either side of the BamHI site of pPL-VP1-1. These plasmids ("pFMDV-1034-Bal") were used to transform E. coli M5219 as described previously. Four hundred colonies were selected and tested for the production of polypeptides having the specificity of FMD viral antigens by Broome-Gilbert assay. Sizing of the plasmids from the positive colonies confirmed that the plasmids were up to 400 nucleotides smaller than the starting pPL-VP1-1. In addition, the fusion proteins produced by these positive colonies were up to 130-150 amino acids smaller than the corresponding proteins produced by hosts transformed with pPL-VP1-1.

This series of Bal 31 deletions confirms that the antigenic portion of VP1 is located near the COOH terminus of VP1 because the Bal 31 deletions have removed 60 to 75 amino acids from the $NH_2$ terminal end of VP1 without destroying the antigenicity of the protein. In addition, it demonstrates that DNA sequences of this invention which contain only a part of VP1 may be usefully employed to produce products having the specificity of FMD viral antigens by expression in appropriate hosts. In addition, the nucleotide sequences of this invention provide previously unavailable information about the amino acid sequences of products which display the specificity of FMD viral antigens. This information then allows the chemical synthesis of small peptide fragments whose amino acid sequences correspond to those sequences coded for by the nucleotide sequences of the antigenic DNA fragment. These synthetic proteins may then be used in vaccines and methods for protecting animals for at least some period of time from FMDV. It is to be understood that both of these processes and products are part of this invention.

The plasmids having the Bal 31 deletions have also been used as a source of VP1 related DNA sequences for insertion into other cloning vectors and hosts. For example, one of the Bal 31 deleted plasmids ("FMDV-1034-Bal-1") was digested with EcoRI and HindIII and the resultant EcoRI-HindIII fragment inserted into pBR325 replacing the EcoRI-HindIII fragment therein. In that vector, the transcription of the hybrid gene is under the control of the chloramphenicol acetyltransferase gene promoter. After transformation of E. coli M5219 with the resultant hybrid plasmid and culturing of the transformed cells, positive colonies producing VP1 related antigens were observed in the usual assays.

Finally, although the above example is concerned principally with manipulations of FMDV-1034, this invention is not so limited. Instead, other FMDV DNA containing clones may be similarly employed to produce hybrid plasmids which in appropriate bacterial hosts will permit the improved expression of the antigenic polypeptides coded for by those DNA sequences. For example, other FMDV-related DNA sequences such as FMDV-144, FMDV-715, FMDV-1824, FMDV-1933, or DNA sequences hybridizing to them may be treated in a similar fashion to that described for FMDV-1034 to improve the yield and activity of the polypeptides coded for by them.

For example, E. coli FMDV-1448 (Figure 3), one of the clones isolated from the initial library of clones was used, as above, to isolate pFMDV-1448 and DNA sequence FMDV-1448. This DNA sequence was then employed to determine the nucleotide sequence of VP3 in a manner similar to that employed in determining the nucleotide sequence of VP1 from FMDV-1034. Referring now to Figure 11, the presently determined nucleotide sequence (and its corresponding amino acid sequence) of VP3 is displayed.

The amino acid sequence determined for VP3 by nucleotide sequencing of FMDV-1448 corresponds well with that determined by protein sequencing for the first 32 amino acids of authentic VP3 by K. Strohmaier et al., supra. From that sequence, it appears that FMDV-1448 begins at amino acid 9 (Asp) of VP3 and continues into VP1, amino acid 221 (Thr) of Figure 11 having been determined to be the first amino acid of VP1 (Figures 3 and 9). The single difference between the amino acid sequence of amino acids 9 to 32 of FMDV-1448 and authentic VP3 occurs at amino acid 23 where nucleotide sequencing predicts Asp and protein sequencing afforded Thr. In addition, the nucleotide at position 310 has not been determined as yet. Accordingly, the amino acid at position 121 may be Phe, Leu, Ile or Val. It is uncertain where VP3 ends since there is no stop codon before the beginning of VP1.

Another example of such manipulations is the production of VP1 together with at least one of VP3, VP2 or VP4 of FMDV Type $O_1K$ so as to make a combined antigenic product. More preferably, the primary product P88 (see Figure 1) is produced in an appropriate host using the DNA sequences, recombinant DNA molecules and processes of this invention to provide a primary FMDV antigenic product useful in vaccines and methods of treating animals.

## USE OF THE ANTIGENIC POLYPEPTIDES OF THIS INVENTION IN IMMUNOGENIC COMPOSITIONS IN VIVO

The several immunoassays described above demonstrate that the DNA sequences and recombinant DNA molecules of this invention direct the production of antigenic polypeptides to FMDV. DNA sequencing has determined that some of these recombinant DNA molecules contain inserts having at least a major portion of structural gene which codes for capsid protein VP1.

Other of these clones have the complete sequence of VP1 and the complete or partial sequences of VP2 and VP3. These data coupled with the known utility of capsid protein VPI in vaccines against FMDV (Bachrach et al., supra) suggest that the antigenic polypeptides produced in accordance with this invention are useful in vaccines against FMDV. In vivo testing of the antigenic polypeptides of this invention has been carried out. As expected the results depend on the amount and purity of the antigenic product produced by the host, but they confirm the utility of the polypeptides and processes of this invention.

For example, the unpurified extract of E. coli NF1($\lambda N^{-}cro^{-}cI_{ts}$-pPL-VP1-1) was injected into mice. Serum prepared from these mice gave weakly positive results in assays for the presence of FMD viral antibodies. In addition, the protein band at ~45,000 d from the bacteria extract of E. coli NF1($\lambda N^{-}cro^{-}cI_{ts}$-pPL-VP1-1) was separated on a polyacrylamide gel. The resultant band was removed from the plate, crushed and used to inject one rabbit and five mice. After a series of three immunizations, serum from these test animals was analyzed for the presence of antibodies to FMDV. The mice serum gave positive results in direct binding with protein sepharose-$^{125}$I-labelled virus and in radioimmune competition assays. However, negative results were observed in enzyme-linked sorbant assay (ELISA) and the serum did neutralize active virus, perhaps owing to a low antibody titer. Negative results in all assays were observed for the rabbit serum.

Finally, the supernatant from a cell extract (as above) of approximately $10^{11}$ bacteria/ml was purified about 10-fold by excessive treatment with nucleases and chromatography on G100 Sephadex. The starting extract and the G-100 peak were then employed to inject three goats. Each goat was injected with three different preparations: extract-Freunds adjuvant, extract-cγ-gel and extract-crosslinked with glutaraldehyde. After three immunizations, two of the goats showed a postive-immune reaction (ELISA). Therefore, the immunogencity of the bacterial-produced FMDV-related antigens of this invention has been demonstrated in animals that are susceptible to FMDV infections. Owing presumably to the low-antibody titer, neutralization of active virus was not observed. Immunization with more purified antigen is expected to produce sera in animals that will neutralize active FMDV.

Therefore, the products made by the processes of these invention are active in vivo to produce antibodies having the specificty of FMD viral antibodies. It should, of course, be understood that the products made by the processes of this invention need not be used alone to vaccinate animals. Instead, the products may be usefully combined physically, chemically or by modified construction of the DNA sequences that code for them with other well known compounds that enhanced the immunological effect and duration of the vaccine.

## IDENTIFICATION OF OTHER PROTEINS DISPLAYING THE SPECIFICITY OF VIRAL ANTIGENS OF FMDV

The methods and procedures of this invention are, of course, not limited to the identification and use of DNA sequences and recombinant DNA molecules characterized by at least a portion of the DNA sequence that codes for capsid protein VP1 or the other antigenic proteins of FMDV, type O. Rather, these methods and procedures may be employed in like manner, by those of skill in the art to select and to use other DNA sequences and recombinant DNA molecules which in appropriate hosts direct the production of other polypeptides that display the specificity of FMDV antigens without departing from the scope of this invention. E.g., DNA sequences and recombinant DNA molecules characterized by a combination of DNA sequences that code for several antigenic proteins of a single FMDV serotype, DNA sequences and recombinant DNA molecules characterized by DNA sequences that code for antigenic proteins of other FMDV serotypes, DNA sequences and recombinant DNA molecules characterized by a combination of DNA sequences that code for various combinations of antigenic polypeptides of more than one or all FMDV serotypes, and DNA sequences and recombinant DNA molecules characterized by DNA sequences that code for a single broad spectrum antigenic polypeptide of several FMDV serotypes.

For example, the methods of this invention maybe employed to isolate the mRNA from other FMDV serotypes and that mRNA analysized and cloned through its cDNA as described in this invention to produce products displaying antigenicity to those serotypes. These products may then be used alone in univalent vaccines or together with products related to the other FMDV serotypes in bivalent or broader spectrum vaccines.

More preferably, the DNA sequences and recombinant DNA molecules of this invention are used directly to afford the preparation DNA sequences and recombinant DNA molecules that direct the production in appropriate hosts of polypeptides displaying the specificity of FMD viral antigens from other FMDV serotypes. This direct process is possible because it has now been determined that the mRNAs of the other FMD viral serotypes are quite homologous to the mRNA of subtype $0_1K$. This homology is also found in the area of the capsid protein related regions of the mRNAs. Therefore, the DNA sequences and recombinant DNA molecules of this invention may be employed to direct the synthesis of DNA sequences from the other FMDV serotypes.

In broad outline this process is characterized by the preparation from FMDV-1034 or other sequence of a series of small "priming" fragments located near the start of the antigenic region of VPl. These fragments are then used to locate by hybridization the corresponding homologous region in the mRNAs from other FMDV serotypes. Once this desired region of the mRNA has been located a cDNA copy of it is made using the hybridized fragment as a "primer". The cDNA may then be used, as described above, to construct recombinant DNA molecules and to produce the desired antigenic polypeptide in appropriate hosts. Alternatively, the nucleotide sequence and therefore the corresponding amino acid sequence of the desired region of the mRNA or cDNA can be determined and that sequence used to prepare the desired antigenic product by traditional chemical methods for subsequent use in FMDV vaccines and treatments.

For example, referring now to Figure 12, FMDV-1034 was cloned into fd (in order to single-strand the double-stranded FMDV-1034 DNA) in an orientation that permitted the production of the minus DNA strand of FMDV-1034. The minus DNA strand was then restricted with HaeIII (see Figure 12) and the resulting several small fragments ("FMDV-1034 (<u>Hae</u>III)") labelled with $^{32}$-P using standard procedures. These fragments were then hybridized to the total mRNA, isolated as described above from another FMDV serotype, e.g., serotype A, in Figure 12. Hybridization allows the area just preceding the VPl antigenic related portion of the mRNA to be located. Then, the hybridized fragment was used as a "primer" to prepare cDNA from the respective RNA regions with reverse transcriptase (Figure 12). The cDNA was then double-stranded as before with DNA polymerase and inserted into an appropriate host/vector combination to produce products displaying the antigenicity of viral antigens of FMDV type A. Alternatively, the regions of the mRNA located by hybridization to the labelled fragment or the cDNA copy thereof were sequenced and that sequence used to prepare peptides by traditional chemical methods that display the antigenicity of the viral antigens of those serotypes. Therefore, the methods and products of this invention are useful not only in the production of products displaying the antigenicity of FMD Type $O_1K$ viral antigens, but also in methods to produce antigens to other FMDV strains.

Micro-organisms and recombinant DNA molecules prepared by the processes described herein are exemplified by cultures deposited in the culture collection of the American Type Culture Collection in Rockville, Maryland on May 5, 1980 and identified as FMDV-A to C.

A: E. coli HB101 (pBR322(Pst)/FMDV-715)

B: E. coli HB101 (pBR322(Pst)/FMDV-331)

C: E. coli HB101 (pBR322(Pst)/FMDV-144)

These cultures were assigned accession numbers ATCC 31640, 31641 and 31642, respectively.

Micro-organisms and recombinant DNA molecules prepared by the processes described herein are also exemplified by cultures deposited in the culture collection of Deutsche Sammlung Von Mikroorganismen in Gottingen, West Germany on May 12, 1980 and identified as FMDV-D to G.

D: E. coli HB101 (pBR322(Pst)/FMDV-703)

E: E. coli HB101 (pBR322(Pst)/FMDV-1034)

F: E. coli HB101 (pBR322(Pst)/FMDV-1824)

G: E. coli HB101 (pBR322(Pst)/FMDV-1933)

These cultures have been assigned accession numbers DSM 1814-1817, respectively. And, micro-organisms and recombinant DNA molecules prepared by the processes described herein, are exemplified by cultures deposited in the culture collection of Deutsche Sammlung von Mikroorganismen in Gottingen, West Germany on July 31, 1980 and identified as FMDV-A to C.

A: E. coli W6 ($\lambda_{rex}$-pPL-VP1-1)

B: E. coli NF1 ($\lambda N^{-}cro^{-}cI_{ts}$-pPL-VP1-1)

C: E. coli NF1 ($\lambda N^{-}cro^{-}cI_{ts}$-pPL-VP1-5)

These cultures have been assigned accession numbers DSM 1879-1881, respectively.

While we have hereinbefore presented a number of embodiments of this invention, it is apparent that our basic construction can be altered to provide other embodiments which utilize the processes and compositions of this invention. Therefore, it will be appreciated that the scope of this invention is to be defined by the claims appended hereto rather than the specific embodiments which have been presented hereinbefore by way of example.

CLAIMS

1. A DNA sequence characterized in that at least a portion thereof codes for a polypeptide displaying FMDV antigenicity and being selected from the group consisting of (a) FMDV-715, FMDV-144, FMDV-1034, FMDV-1448, FMDV-1824, FMDV-1933, (b) DNA sequences which hybridize to any of the foregoing DNA sequences, (c) DNA sequences, from whatever source obtained, including natural, synthetic or semi-synthetic sources, related by mutation, including single or multiple, base substitutions, deletions, insertions and inversions to any of the foregoing DNA sequences, and (d) DNA sequences comprising sequences of codons which code for an antigenic polypeptide containing an amino acid sequence similar to those coded for by codons of any of the foregoing DNA sequences.

2. A DNA sequence according to claim 1 characterized in that the DNA sequence (b) which hybridizes to at least one of the DNA sequences (a) is selected from the group consisting of (e) VP1-1, VP1-5, FMDV-1034-Bal, FMDV-1034-Bal-1(EcoRI-HindIII), (f) DNA sequences which hybridize to any of the foregoing DNA sequences, (g) DNA sequences, from whatever source obtained, including natural, synthetic or semi-synthetic sources, related by mutation, including single or multiple, base substitutions, deletions, insertions and inversions to any of the foregoing DNA sequences, and (h) DNA sequences comprising sequences of codons which code for an antigenic polypeptide containing an amino acid sequence similar to those coded for by the codons of any of the foregoing DNA sequences.

3. A DNA sequence according to claim 1 or 2 characterized in that the DNA sequences (b) or (f) which hybridizes to at least one of the DNA sequences (a) or (e) is selected from the group of (i) HaeIII fragments of FMDV-715, FMDV-144, FMDV-1034, FMDV-1448, FMDV-1824, FMDV-1933, VP1-1, VP1-5, FMDV-1034-Bal, FMDV-1034-Bal-1 (EcoRI-HindIII), and (j) DNA sequences which hybridize to any of the foregoing DNA sequences.

4. A DNA sequence according to any one of claims 1 to 3 characterized in that the DNA sequences (b), (f) or (j) which hybridizes to at least one of the DNA sequences (a), (e) or (i) is selected from the group consisting of DNA sequences coding for a single antigenic polypeptide of a single FMDV serotype, DNA sequences coding for a combination of antigenic polypeptides of a single FMDV serotype, DNA sequences coding for a combination of antigenic polypeptides of more than one FMDV serotype and DNA sequences coding for a single antigenic polypeptide for more than one FMDV serotype.

5. A DNA sequence according to claim 4 characterized in that said FMDV serotype is selected from the group consisting of FMDV type O, A, C, SAT 1, SAT 2, SAT 3 and Asian type I.

6. A DNA sequence according to any one of claims 1 to 5 characterized in that it is selected from the group consisting of DNA sequences of the formula:
ACCACTTCTGCGGGCGAGTCAGCGGATCCTGTCACCACCACCGTTGAAAACTACG
GTGGCGAAACACAGATCCAGAGGCGCCAACACACGGACGTCTCGTTCATCATGGA
CAGATTTGTGAAGGTGACACCGCAAAACCAAATTAACATTTTGGACCTCATGCAG
ATTCCATCACACACTTTGGTGGGAGCACTCCTACGCGCGTCCACTTACTACTTCT
CTGACTTGGAGATAGCAGTAAAACACGAGGGAGACCTCACCTGGGTTCCAAATGG
AGCGCCCGAAAAGGCGTTGGACAACACCACCAACCCAACTGCTTACCACAAGGCA
CCACTCACCCGGCTTGCCCTGCCTCACACTGCGCCCCACCGCGTGTTGGCAACCG
TGTACAACGGTGAGTGCAGGTACAACAGAAATGCTGTGCCCAACTTGAGAGGTGA
CCTTCAGGTGTTGGCTCAAAAGGTGGCACGGACGCTGCCTACCTCCTTCAACTAC
GGTGCCATCAAAGCGACCCGGGTCACCGAGTTGCTTTACCGGATGAAGAGGGCCG
AAACATACTGTCCAAGGCCCTTGCTGGCAATCCACCCAACTGAAGCCAGACACAA
ACAGAAAATTGTGGCACCGGTGAAACAGACTTTG and fragments and derivatives thereof, said fragments and derivatives coding for antigenic polypeptides of FMDV.

7. A DNA sequence according to any one of claims 1 to 5 characterized in that it is selected from the group consisting of DNA sequences of the formula:
GCGACGGCTATGGTGGCCTGGTGACCACGGACCCGAAGACGGCTGACCCCGTTT
ATGGGAAAGTGTTCAACCCCCCCCGCAACCAGTTGCCGGGGCGTTTTACCAACC
TCCTTGATGTGGCTGAGGCATGCCCGACGTTTCTGCGCTTCGAGGGTGGCGTAC
CGTACGTGACCACGAAAACGGACTCGGACACCCTACTTGCTCAGTTTGACATGT
CTTTGGCAGCAAAACAAATGTCAAACACCTTCCTCGCAGGTCTTGCGCAGTAC
TACACACAGTACAGTGGCACCATCAACCTGCACTTCATG?TCACAGGACCCACTG
ACGCGAAGGCGCGTTACATGGTTGCCTACGCCCCACTAGGCATGGAGCCGCCCA
AGACACCTGAGGCGGCCGCGCACTGCATTCATGCTGAATGGGACACTGGGTTAA
ACTCAAAGTTTACTTTTTCCATCCCCTACCTCTCGGCCGCCGATTACGCGTACA
CCGCGTCTGGCGTGGCCGAGACCACAAATGTGCAGGGATGGGTCTGCTTGTTTC
AAATTACACATGGCAAGGCCGACGGCGACGCTCTGGTCGTACTGGCTAGTGCTG
GTAAAGACTTTGAGCTAAGGCTGCCGGTGGACGCCCGTGCGGAA and fragment and derivatives thereof, said fragments and derivatives coding for antigenic polypeptides of FMDV.

8. A variant of the DNA sequence according to any one of the preceding claims characterized in that it is selected from the group consisting of FMDV-703, FMDV-331, DNA sequences which hybridize to either of the foregoing DNA sequences, DNA sequences, from whatever source obtained, including natural, synthetic or semi-synthetic sources, related by mutation, including single or multiple, base substitutions, deletions, insertions and inversions to any of the foregoing DNA sequences and DNA sequences comprising sequences of codons which code for antigenic polypeptides containing an amino acid sequence similar to those coded for by the codons of any of the foregoing DNA sequences.

9. A recombinant DNA molecule characterized by a DNA sequence according to any one of the preceding claims.

10. A recombinant DNA molecule according to claim 9, characterized in that said DNA sequence is operatively linked to an expression control sequence.

11. A recombinant DNA molecule according to claim 10 characterized in that the expression control sequence is selected from the group consisting of the E. coli lac system, the E. coli trp system, the major operator and promoter regions of phage λ, the control region of Filamenteous single-stranded DNA phages, the β-lactamase system of E. coli plasmids or other sequences which control the expression of genes of prokaryotic or eukaryotic cells and their viruses.

12. A recombinant DNA molecule according to claim 10 or 11, as those claims depend from claim 1, characterized in that it is selected from the group consisting of pFMDV-715, pFMDV-144, pFMDV-1034, pFMDV-1448, pFMDV-1824 and pFMDV-1933.

13. A recombinant DNA molecule according to claim 10 or 11, as those claims depend from claim 2, characterized in that it is selected from the group consisting of pPL-VP1-1, pPL-VP1-5, pFMDV-1034-Bal and pFMDV-1034-Bal-1(EcoRI-HindIII).

14. A recombinant DNA molecule according to claim 10 or 11 as those claims depend from claim 8, characterized in that it is selected from the group consisting of pFMDV-703 and pFMDV-331.

15. A host transformed with at least one recombinant DNA molecule according to any one of claims 9 to 14.

16. A host according to claim 15 characterized in that it is selected from the group consisting of strains of E. coli, Pseudomonas, Bacillus subtilis, Bacillus stearothermophilus, other bacilli, yeasts, other fungi, animal and plant hosts and human tissue cells.

17. A transformed host according to claim 15 or 16, as those claims depend from claim 12, characterized in that it is selected from the group consisting of E. coli HB101 (pBR322(Pst)/FMDV-715), E. coli HB101 (pBR322(Pst)/FMDV-144), *E. coli* HB1301 (pBR322(Pst)/FMDV-1034), *E. coli* HB101 (pBR322(Pst)/FMDV-1448), *E. coli* HB101 (pBR322(Pst)/FMDV-1824) and *E. coli* HB101 (pBR322(Pst)/FMDV-1933).

18. A transformed host according to claim 15 or 16, as those claims depend from claim 13 characterized in that it is selected from the group consisting of *E. coli* NF1 ($\lambda N^{-}cro^{-}cI_{ts}$-pPL-VP1-1), *E. coli* M5219 ($\lambda N^{+}cro^{-}cI_{ts}$-pPL-VP1-1), *E. coli* W6($\lambda_{rex}$-pPL-VP1-5), [add 1034-Bal, 1034-Bal-1 (*Eco*RI-*Hind*III).

19. A transformed host according to claim 15 or 16, as those claims depend from claim 14, characterized in that it is selected from the group consisting of *E. coli* HB101 (pBR322(Pst)/FMDV-703) and *E. coli* HB101 (pBR322(Pst)/FMDV-331).

20. A polypeptide or fragment or derivative thereof displaying the antigenicity of FMD viral antigens and produced by the transformed host according to any one of claims 15 to 19.

21. A polypeptide characterized in that it is coded for by a DNA sequence according to any one of claims 1 to 8.

22. A polypeptide according to claim 20 or 21 characterized in that the FMD viral antigens are selected from the group of antigens to FMDV serotypes O, A, C, SAT1, SAT2, SAT3, Asian I and combinations thereof.

23. A polypeptide according to claim 20 or 21 as those claims depend from claim 17, characterized in that at least a portion of it is selected from the group consisting of FMDV-715, FMDV-144, FMDV-1034, FMDV-1448, FMDV-1824, FMDV-1933 and fragments and derivatives thereof, said polypeptide displaying the antigenicity of FMD viral antigens.

24. A polypeptide according to claim 20 or 21 as those claims depend from claim 18, characterized in that at least a portion of it is selected from the group consisting of VP-1, VP1-1, VP1-5, FMDV-1034-Bal, FMDV-1034-Bal-1(EcoRI-HindIII), and fragments and derivatives thereof, said polypeptide displaying the antigencity of FMD viral antigens.

25. A polypeptide according to claim 24 characterized in that at least a portion of it is selected from polypeptides of the group consisting of THRTHRSERALAGLYGLUSERALAASPPROVALTHRTHRTHRVALGLUASNTYRGLYGLYGLUTHRGLNILEGLNARGARGGLNHISTHRASPVALSERPHEILEMETASPARGPHEVALLYSVALTHRPROGLNASNGLNILEASNILELEUASPLEUMETGLNILEPROSERHISTHRLEUVALGLYALALEULEUARGALASERTHRTYRTYRPHESERASPLEUGLUILEALAVALLYSHISGLUGLYASPLEUTHRTRYVALPROASNGLYALAPROGLULYSALALEUASPASNTHRTHRASNPROTHRALATYRHISLYSALAPROLEUTHRARGLEUALALEUPROHISTHRALAPROHISARGVALLEUALATHRVALTYRASNGLYGLUCYSARGTYRASNARGASNALAVALPROASNLEUARGGLYASPLEUGLNVALLEUALAGLNLYSVALALAARGTHRLEUPROTHRSERPHEASNTYRGLYALAILELYSALATHRARGVALTHRGLULEULEUTYRARGMETLYSARGALAGLUTHRTYRCYSPROARGPROLEULEUALAILEHISPROTHRGLUALAARGHISLYSGLNLYSILEVALALAPROVALLYSGLNTHRLEU, ASPPROVALTHRTHRTHRVALGLUASNTYRGLYGLYGLUTHRGLNILEGLNARGARGGLNHISTHRASPVALSERPHEILEMETASPARGPHEVALLYSVALTHRPROGLNASNGLNILEASNILELEUASPLEUMETGLNILEPROSERHISTHRLEUVALGLYALALEULEUARGALASERTHRTYRTYRPHESERASPLEUGLUILEALAVALLYSHISGLUGLYASPLEUTHRTRYVALPROASNGLYALAPROGLULYSALALEUASPASNTHRTHRASNPROTHRALATYRHISLYSALAPROLEUTHRARGLEUALALEUPROHISTHRALAPROHISARGVALLEUALATHRVALTYRASNGLYGLUCYSARGTYRASNARGASNALAVALPROASNLEUARGGLYASPLEUGLNVALLEUALAGLNLYSVALALAARGTHRLEUPROTHRSERPHEASNTYRGLYALAILELYSALATHRARGVALTHRGLULEULEUTYRARGMETLYSARGALAGLUTHRTYRCYSPROARGPROLEULEUALAILEHISPROTHRGLUALAARGHISLYSGLNLYSILEVALALAPROVALLYSGLNTHRLEU, ASPPROVALTHRTHRTHRVALGLUASNTYRGLYGLYGLUTHRGLNILEGLNARGARGGLNHISTHRASPVALSERPHEILEMETASPARGPHEVALLYSVALTHRPROGLNASNGLNILEASNILELEUASPLEUMETGLNILEPROSERHISTHRLEUVALGLYALALEULEUARGALASERTHRTYRTYRPHESERASPLEUGLUILEALAVALLYSHISGLUGLYASPLEUTHRTRYVALPROASNGLYALAPROGLULYSALALEUASPASNTHRTHRASNPROTHRALATYRHISLYSALAPROLEUTHRARGLEUALALEUPROHISTHRALAPROHISARGVALLEUALATHRVALTYRASNGLYGLUCYSARGTYRASNARGASN

ALAVALPROASNLEUARGGLYASPLEUGLNVALLEUALAGLNLYSVALALAARGTHR LEUPROTHRSERPHEASNTYRGLYALAILELYSALATHRARGVALTHRGLULEULEU TYRARGMETLYSARGALAGLUTHRTYRCYSPROARGPROLEULEUALAILEHISPRO THRGLUALAARGHISLYSGLNLYSILEVALALALAPROVALLYSGLNTHRLEUASNPHE ASPLEULEULYSLEUALAGLYASPVALGLUSERASNPROGLYPROPHEPHEPHESER ASPVALARGSERASNPHESERLYSLEUVALGLUTHRTHRASNGLNMETGLNGLUASP METSERTHRLYSHISGLYPROASPPHEASNARGLEUVALPHEALAPHEGLUGLULEU ALAILEGLYVALLYSALAILEARGTHRGLYLEUASPGLUALALYSPROTRYTYRLYS LEU and fragments and derivatives thereof, said polypeptide displaying the antigenicity of FMD viral antigens.

26. A polypeptide according to claim 23 characterized in that at best a portion of it is selected from polypeptides of the group consisting of AspGlyTyr GlyGlyLeuValThrThrAspProLysThrAlaAspProValTyrGlyLysVal PheAsnProProArgAsnGlnLeuProGlyArgPheThrAsnLeuLeuAspVal AlaGluAlaCysProThrPheLeuArgPheGluGlyGlyValProTyrValThr ThrLysThrAspSerAspThrLeuLeuAlaGlnPheAspMetSerLeuAlaAla LysGlnMetSerAsnThrPheLeuAlaGlyLeuAlaGlnTyrTyrThrGlnTyr SerGlyThrIleAsnLeuHisPheMet(?)ThrGlyProThrAspAlaLysAla ArgTyrMetValAlaTyrAlaProLeuGlyMetGluProProLysThrProGlu AlaAlaAlaHisCysIleHisAlaGluTrpAspThrGlyLeuAsnSerLysPhe ThrPheSerIleProTyrLeuSerAlaAlaAspTyrAlaTyrThrAlaSerGly ValAlaGluThrThrAsnValGlnGlyTrpValCysLeuPheGlnIleThrHis GlyLysAlaAspGlyAspAlaLeuValValLeuAlaSerAlaGlyLysAspPhe GluLeuArgLeuProValAspAlaArgAlaGlu and fragments and derivatives thereof, said polypeptide displaying the antigenicity of FMD viral antigens.

27. A polypeptide according to claim 20 or 21 as those claims depend from claim 19, characterized in that at least a portion of it is selected from the group consisting of FMDV-703, FMDV-331 and fragments and derivatives thereof, said polypeptide displaying the antigenicity of FMD viral antigens.

28. A method for producing a recombinant DNA molecule characterized by the step of introducing into a cloning vehicle a DNA sequence according to any one of claims 1 to 8.

29. A method according to claim 27 characterized by the additional step of introducing into said cloning vehicle an expression control sequence according to claim 11, said expression control sequence being introduced into said cloning vehicle so as to control and to regulate the expression of said DNA sequence.

30. A method for transforming a host characterized by the step of introducing into a host a recombinant DNA molecule according to any one of claims 9 to 14.

31. A method for producing a polypeptide displaying the specificity of FMD viral antigens comprising the steps of transforming an appropriate host with a recombinant DNA molecule according to any one of claims 10 to 14; culturing said host; and collecting said polypeptide.

32. The method according to claim 31, characterized in that the host is selected from the group consisting of strains of E. coli, Pseudomonas, Bacillus subtilis, Bacillus stearothermophilus, other bacilli, yeasts, fungi, animal or plant hosts, and human tissue cells.

33. A method for producing a polypeptide displaying the specificity of FMD viral antigens comprising the steps of culturing a host transformed by a recombinant DNA molecule according to any one of claims 10 to 14 and collecting said polypeptide.

34. A process for selecting a DNA sequence coding for a polypeptide displaying the specificity of FMD viral antigens from a group of DNA sequences, characterized by the step of screening the DNA sequences to determine which hybridize to the DNA sequences according to any one of claims 1 to 8.

35. The process of claim 34 characterized in that the DNA sequence screened is selected from the group consisting of DNA sequences from natural sources, synthetic DNA sequences, DNA sequences from recombinant DNA molecules and DNA sequences which are a combination of any of the foregoing DNA sequences.

36. The process of claim 34 or 35 characterized in that the DNA sequence screened is selected from the group consisting of cDNA sequences from RNA of FMDV type O, A, C, SAT1, SAT2, SAT3 and Asian I.

37. The process of any one of claims 34 to 36, as they depend from claim 3, characterized by the further step of using the hybridized DNA sequence as a primer either to permit at least a portion of the adjacent unhybridized portion of the DNA to be transcribed by reverse transcriptase or to permit at least a portion of the adjacent unhybridized portion of the DNA to be sequenced.

38. A composition for rendering cloven-footed animals resistant for at least some time to RMDV comprising at least one polypeptide according to any one of claims 20 to 27.

39. A composition according to claim 38 further characterized in that it comprises a pharmaceutically acceptable carrier.

40. A method of treating cloven-footed animals to render them resistant for at least some time to FMDV comprising treating them in a pharmaceutically acceptable manner with a composition according to claim 38 or 39.

FIG. 1

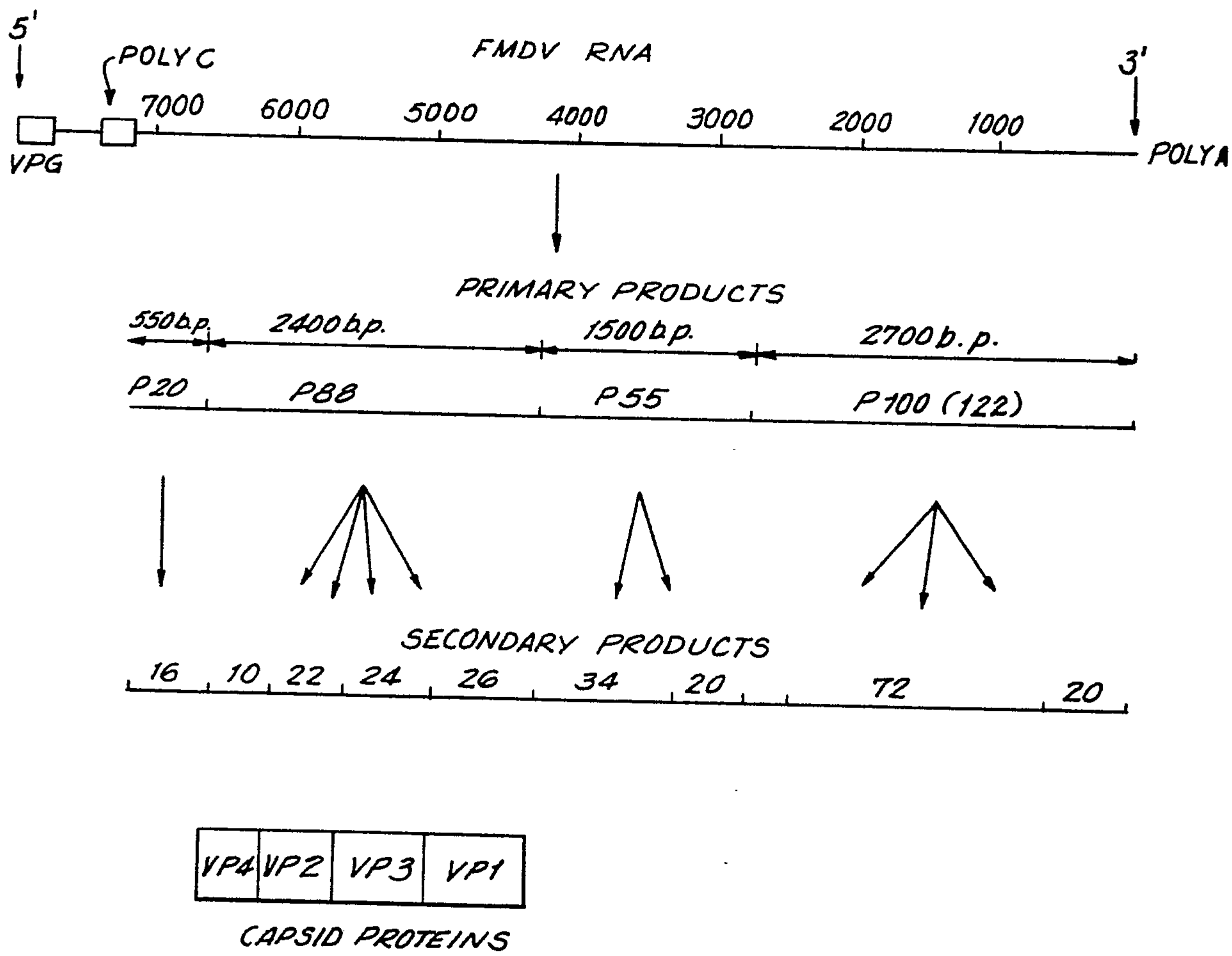
5'
POLY C
FMDV RNA
3'
7000
6000
5000
4000
3000
2000
1000
VPG
POLY A
PRIMARY PRODUCTS
550 b.p.
2400 b.p.
1500 b.p.
2700 b.p.
P20
P88
P55
P100 (122)
SECONDARY PRODUCTS
16
10
22
24
26
34
20
72
20
VP4
VP2
VP3
VP1
CAPSID PROTEINS

FIG. 2

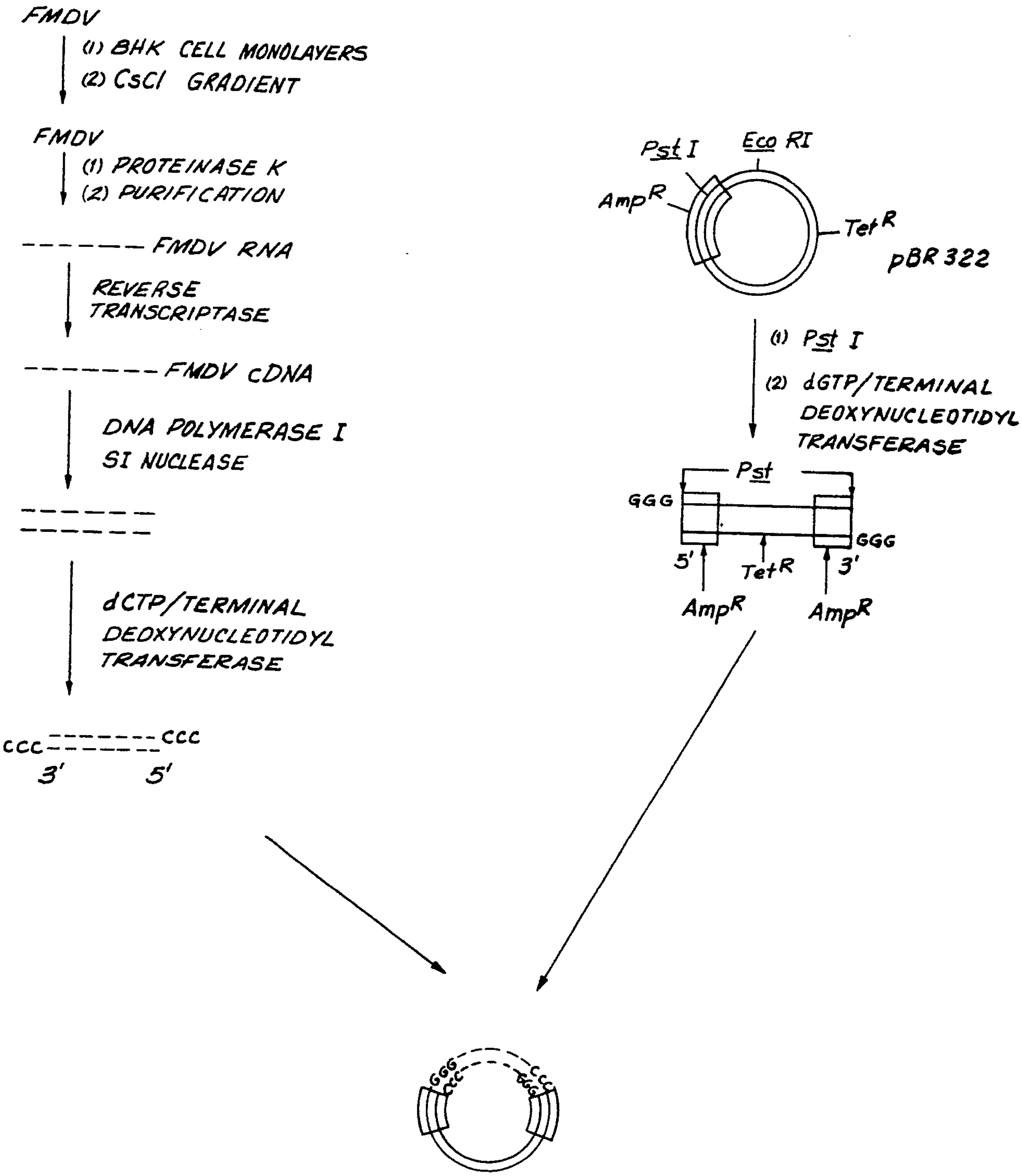


pBR 322 - FMDV

FIG.3

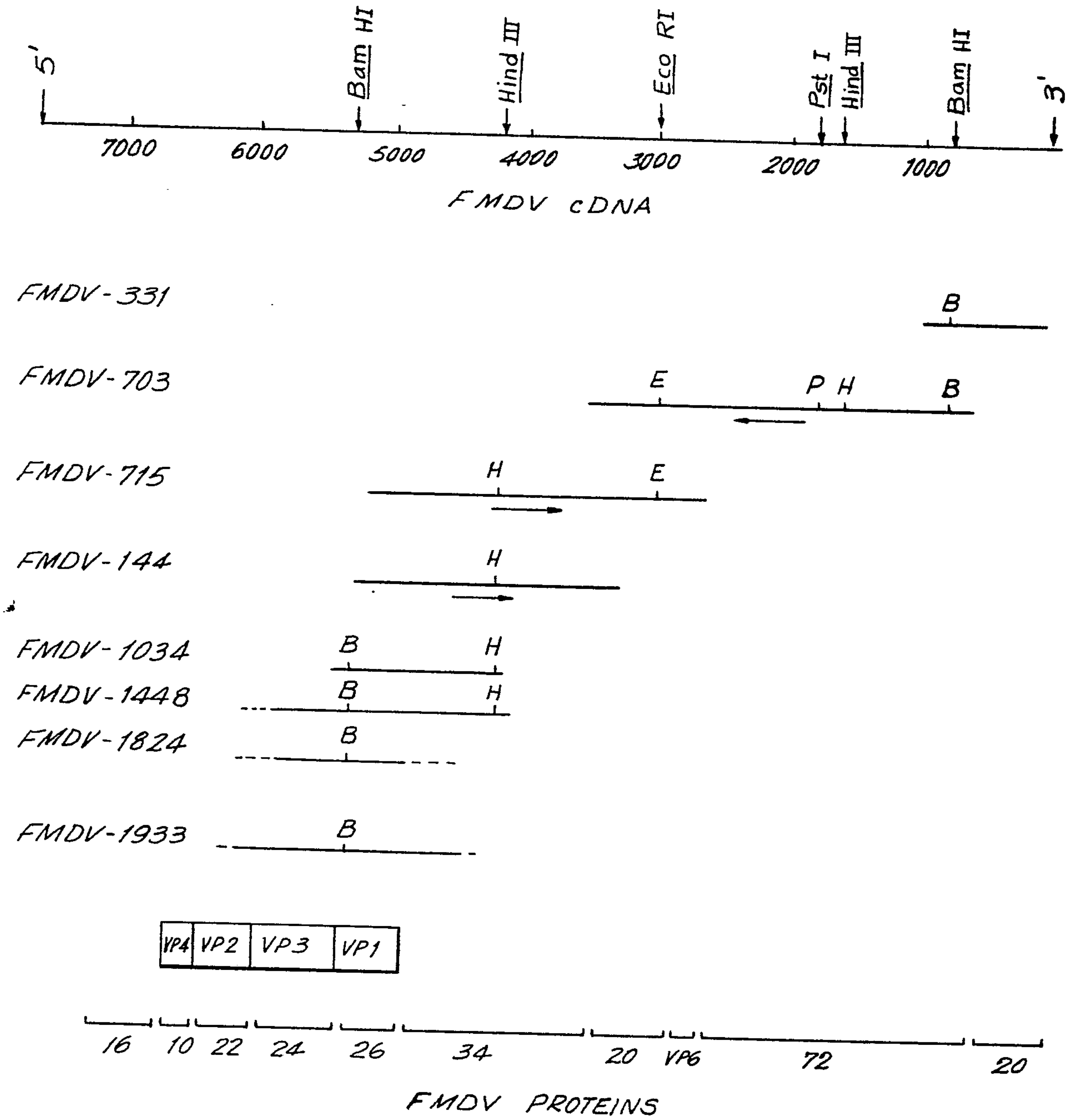
5'
Bam HI
Hind III
Eco RI
Pst I
Hind III
Bam HI
3'
7000
6000
5000
4000
3000
2000
1000
FMDV cDNA
FMDV-331
B
FMDV-703
E
P H
B
FMDV-715
H
E
FMDV-144
H
FMDV-1034
B
H
FMDV-1448
B
H
FMDV-1824
B
FMDV-1933
B
VP4
VP2
VP3
VP1
16
10
22
24
26
34
20
VP6
72
20
FMDV PROTEINS

# FIG. 4

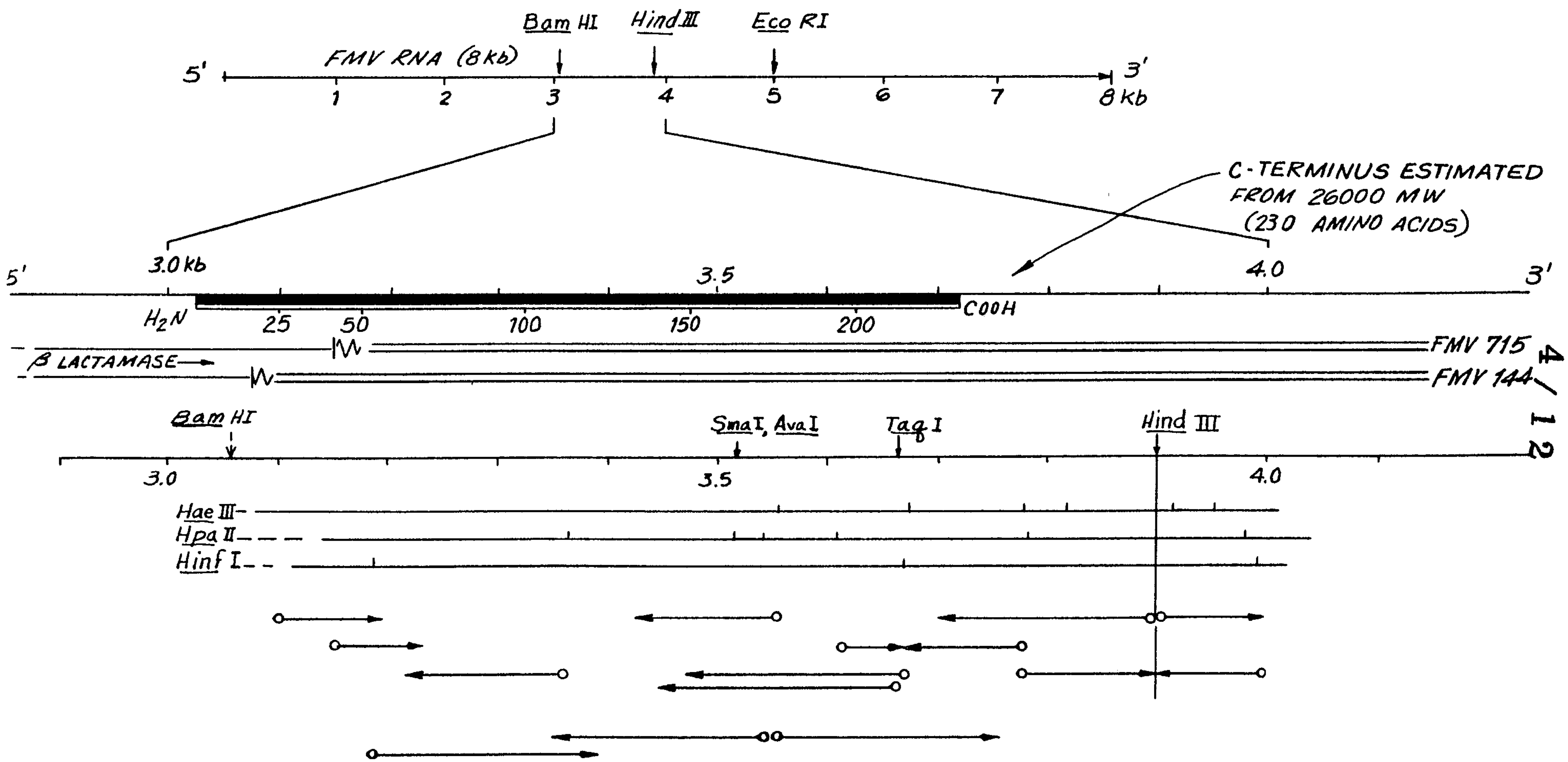

Bam HI
Hind III
Eco RI
5'
FMV RNA (8kb)
3'
1
2
3
4
5
6
7
8 kb
C-TERMINUS ESTIMATED FROM 26000 MW (230 AMINO ACIDS)
5'
3.0 kb
3.5
4.0
3'
H2N
25
50
100
150
200
COOH
β LACTAMASE
FMV 715
FMV 144
Bam HI
Sma I, Ava I
Taq I
Hind III
3.0
3.5
4.0
Hae III
Hpa II
Hinf I

EMDV - 144

| | |
|---|---|
| NUCLEOTIDE SEQUENCE | GGGGGGGGGCAGATCCAGAGGCGCCAACACACGGACGTCTCGTTCATCATGAACAGATTTGTT---- |
| AMINO ACID SEQUENCE | GlyGlyGlyGlnIleGlnArgArgGlnHisThrAspValSerPheIleMetAsnArgPheVal---- |

FIG. 5

FIG.6

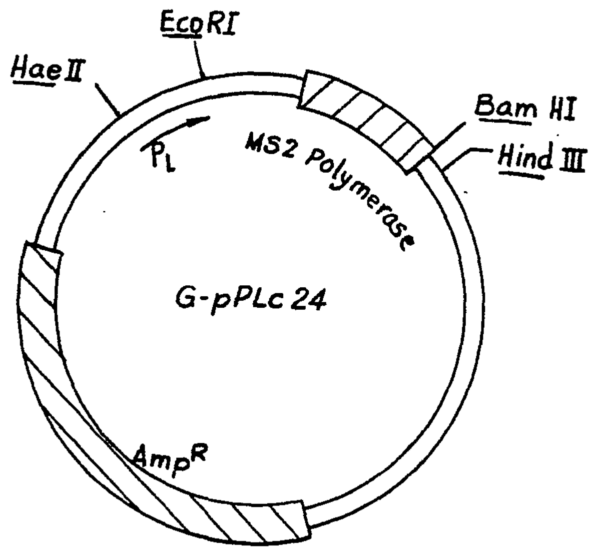


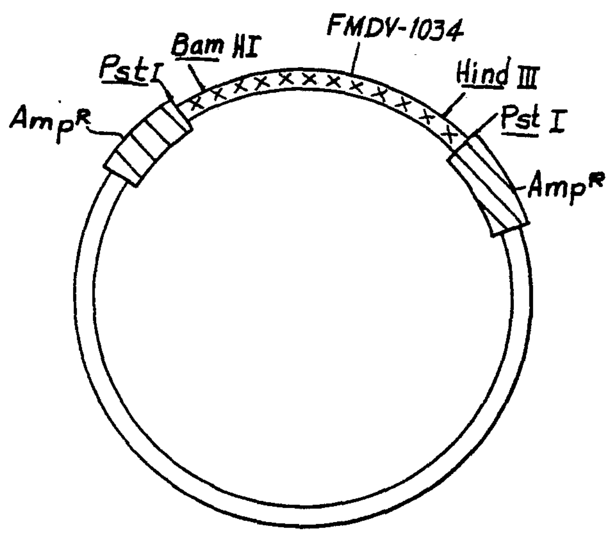


pBR 322 (Pst)/FMDV-1034
pFMDV-1034

FIG.7

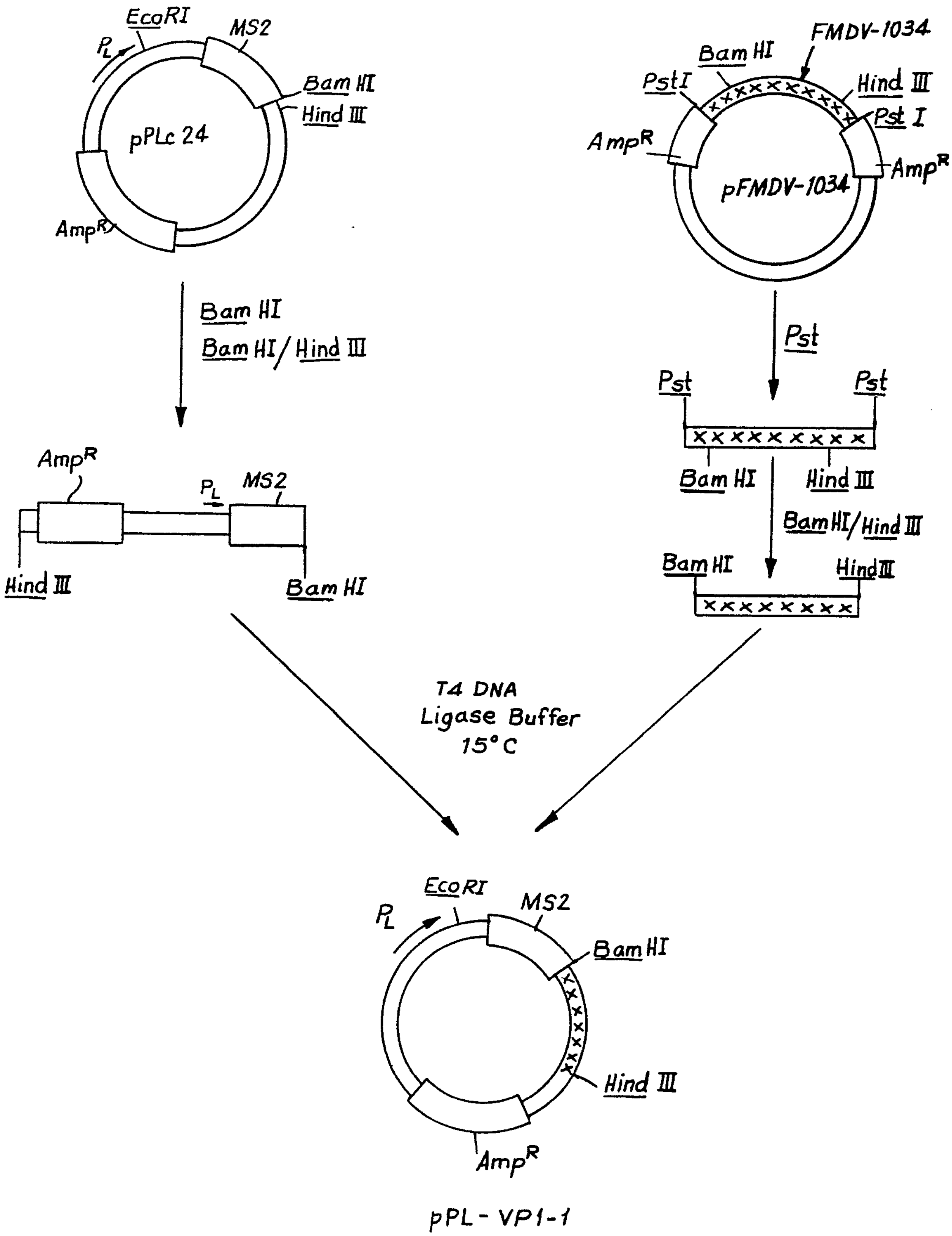
EcoRI
MS2
$P_L$
Bam HI
Hind III
pPLc 24
AmpR
FMDV-1034
Bam HI
PstI
Hind III
Pst I
AmpR
pFMDV-1034
AmpR
Bam HI
Bam HI/Hind III
Pst
Pst
Pst
Bam HI
Hind III
Bam HI/Hind III
Bam HI
Hind III
AmpR
$P_L$
MS2
Hind III
Bam HI
T4 DNA
Ligase Buffer
15°C
EcoRI
MS2
$P_L$
Bam HI
Hind III
AmpR
pPL-VP1-1

FIG. 8

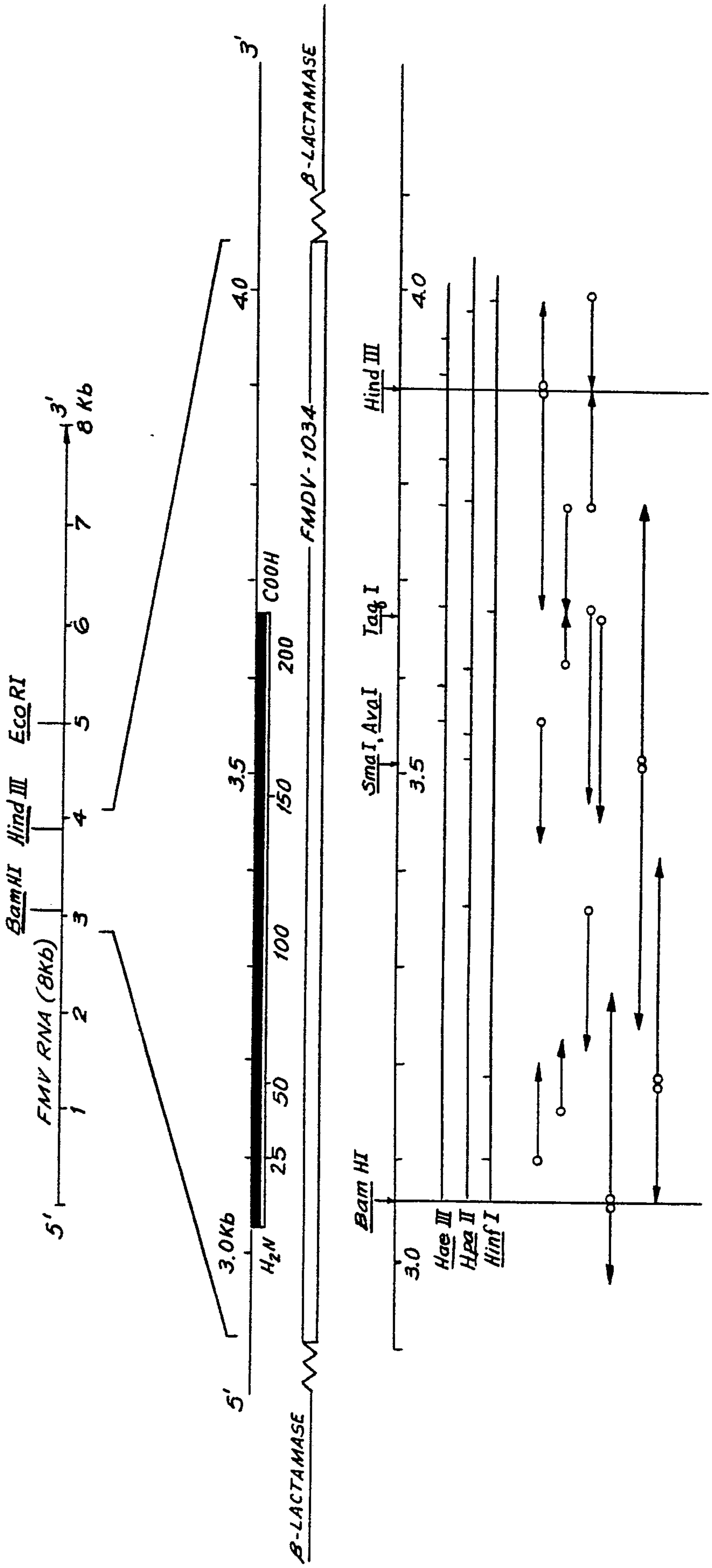
FMV RNA (8Kb)
5'
1
2
3
4
5
6
7
3'
8 Kb
BamHI
Hind III
EcoRI
5'
3.0Kb
3.5
4.0
3'
H2N
25
50
100
150
200
COOH
B-LACTAMASE
FMDV-1034
B-LACTAMASE
Bam HI
Sma I, Ava I
Taq I
Hind III
3.0
3.5
4.0
Hae III
Hpa II
Hinf I

```
-99  .........................................CGTACTGGCTAGTGCTGGTAAAGACTTTGAGCTAAGGCTGCCGGTGGACGCCCGTGCGGAA
                                                 ValLeuAlaSerAlaGlyLysAspPheGluLeuArgLeuProValAspAlaArgAlaGlu
                       BamHI
  1  ACCACTTCTGCGGGCGAGTCAGCGGATCCTGTCACCACCACCGTTGAAAACTACGGTGGCGAAACACAGATCCAGAGGCGCCAACACACGGACGTCTCGT
                              10                            20                            30
     ThrThrSerAlaGlyGluSerAlaAspProValThrThrThrValGluAsnTyrGlyGlyGluThrGlnIleGlnArgArgGlnHisThrAspValSerPhe

101  TCATCATGGACAGATTTGTGAAGGTGACACCGCAAAACCAAATTAACATTTTGGACCTCATGCAGATTCCATCACACACTTTGGTGGGAGCACTCCTACG
                    40                            50                            60
      IleMetAspArgPheValLysValThrProGlnAsnGlnIleAsnIleLeuAspLeuMetGlnIleProSerHisThrLeuValGlyAlaLeuLeuArg

201  CGCGTCCACTTACTACTTCTCTGACTTGGAGATAGCAGTAAAACACGAGGGAGACCTCACCTGGGTTCCAAATGGAGCGCCCGAAAAGGCGTTGGACAAC
           70                            80                            90                           100
     AlaSerThrTyrTyrPheSerAspLeuGluIleAlaValLysHisGluGlyAspLeuThrTryValProAsnGlyAlaProGluLysAlaLeuAspAsn

301  ACCACCAACCCAACTGCTTACCACAAGGCACCACTCACCCGGCTTGCCCTGCCTCACACTGCGCCCCACCGCGTGTTGGCAACCGTGTACAACGGTGAGT
                              110                           120                           130
     ThrThrAsnProThrAlaTyrHisLysAlaProLeuThrArgLeuAlaLeuProHisThrAlaProHisArgValLeuAlaThrValTyrAsnGlyGluCys

401  GCAGGTACAACAGAAATGCTGTGCCCAACTTGAGAGGTGACCTTCAGGTGTTGGCTCAAAAGGTGGCACGGACGCTGCCTACCTCCTTCAACTACGGTGC
                    140                           150                           160
      ArgTyrAsnArgAsnAlaValProAsnLeuArgGlyAspLeuGlnValLeuAlaGlnLysValAlaArgThrLeuProThrSerPheAsnTyrGlyAla

501  CATCAAAGCGACCCGGGTCACCGAGTTGCTTTACCGGATGAAGAGGGCCGAAACATACTGTCCAAGGCCCTTGCTGGCAATCCACCCAACTGAAGCCAGA
           170                           180                           190                           200
     IleLysAlaThrArgValThrGluLeuLeuTyrArgMetLysArgAlaGluThrTyrCysProArgProLeuLeuAlaIleHisProThrGluAlaArg

601  CACAAACAGAAAATTGTGGCACCGGTGAAACAGACTTTGAATTTTGACCTTCTCAAGTTGGCGGGAGACGTCGAGTCCAACCCTGGGCCCTTCTTTTTCT
                205           210                           220                           230
     HisLysGlnLysIleValAlaProValLysGlnThrLeuAsnPheAspLeuLeuLysLeuAlaGlyAspValGluSerAsnProGlyProPhePhePheSer
```

FIG. 9

```
701  CCGACGTTAGGTCGAACTTCTCCAAACTGGTGGAAACCACCAACCAGATGCAGGAGGACATGTCAACAAAACACGGGCCTGACTTTAACCGGTTAGTGTT
                           240                                         250                                   260
      AspValArgSerAsnPheSerLysLeuValGluThrThrAsnGlnMetGlnGluAspMetSerThrLysHisGlyProAspPheAsnArgLeuValPhe

801  CGCATTTGAGGAGTTGGCCATTGGAGTGAAAGCCATCAGAACCGGTCTCGACGAAGCCAAACCCTGGTACAAGCTTATCAAGCTCCTAAGCCGCCTGTCG
              270                                280                               290                        300
     AlaPheGluGluLeuAlaIleGlyValLysAlaIleArgThrGlyLeuAspGluAlaLysProTryTyrLysLeuIleLysLeuLeuSerArgLeuSer
                                                                                  HindIII
```

# FIG. 10

# FIG. 11

1 .......................GCGACGGCTATGGTGGCCTGGTGACCACGGACCCGAAGACGGCTGACCCCGTTTATGGGAAAGTGTTCAACCCCCCC
10 20 30
*GlyIlePheProValAla(?)(?)AspGlyTyrGlyGlyLeuValThrThrAspProLysThrAlaAspProValTyrGlyLysValPheAsnProProArg*

101 GCAACCAGTTGCCGGGGCGTTTTACCAACCTCCTTGATGTGGCTGAGGCATGCCCGACGTTTCTGCGCTTCGAGGGTGGCGTACCGTACGTGACCACGAA
40 50 60
*AsnGlnLeuProGlyArgPheThrAsnLeuLeuAspValAlaGluAlaCysProThrPheLeuArgPheGluGlyGlyValProTyrValThrThrLys*

201 AACGGACTCGGACACCCTACTTGCTCAGTTTGACATGTCTTTGGCAGCAAAACAAATGTCAAACACCTTCCTCGCAGGTCTTGCGCAGTACTACACACAG
70 80 90 100
*ThrAspSerAspThrLeuLeuAlaGlnPheAspMetSerLeuAlaAlaLysGlnMetSerAsnThrPheLeuAlaGlyLeuAlaGlnTyrTyrThrGln*

301 TACAGTGGCACCATCAACCTGCACTTCATG?TCACAGGACCCACTGACGCGAAGGCGCGTTACATGGTTGCCTACGCCCCACTAGGCATGGAGCCGCCCA
110 120 130
*TyrSerGlyThrIleAsnLeuHisPheMet(?)ThrGlyProThrAspAlaLysAlaArgTyrMetValAlaTyrAlaProLeuGlyMetGluProProLys*

401 AGACACCTGAGGCGGCCGCGCACTGCATTCATGCTGAATGGGACACTGGGTTAAACTCAAAGTTTACTTTTTCCATCCCCTACCTCTCGGCCGCCGATTA
140 150 160
*ThrProGluAlaAlaAlaHisCysIleHisAlaGluTrpAspThrGlyLeuAsnSerLysPheThrPheSerIleProTyrLeuSerAlaAlaAspTyr*

501 CGCGTACACCGCGTCTGGCGTGGCCGAGACCACAAATGTGCAGGGATGGGTCTGCTTGTTTCAAATTACACATGGCAAGGCCGACGGCGACGCTCTGGTC
170 180 190 200
*AlaTyrThrAlaSerGlyValAlaGluThrThrAsnValGlnGlyTrpValCysLeuPheGlnIleThrHisGlyLysAlaAspGlyAspAlaLeuVal*

601 GTACTGGCTAGTGCTGGTAAAGACTTTGAGCTAAGGCTGCCGGTGGACGCCCGTGCGGAAACCACTTCTGCGGGCGAGTCAGCG
210 220
*ValLeuAlaSerAlaGlyLysAspPheGluLeuArgLeuProValAspAlaArgAlaGluThrThrSerAlaGlyGluSer*

FIG. 12

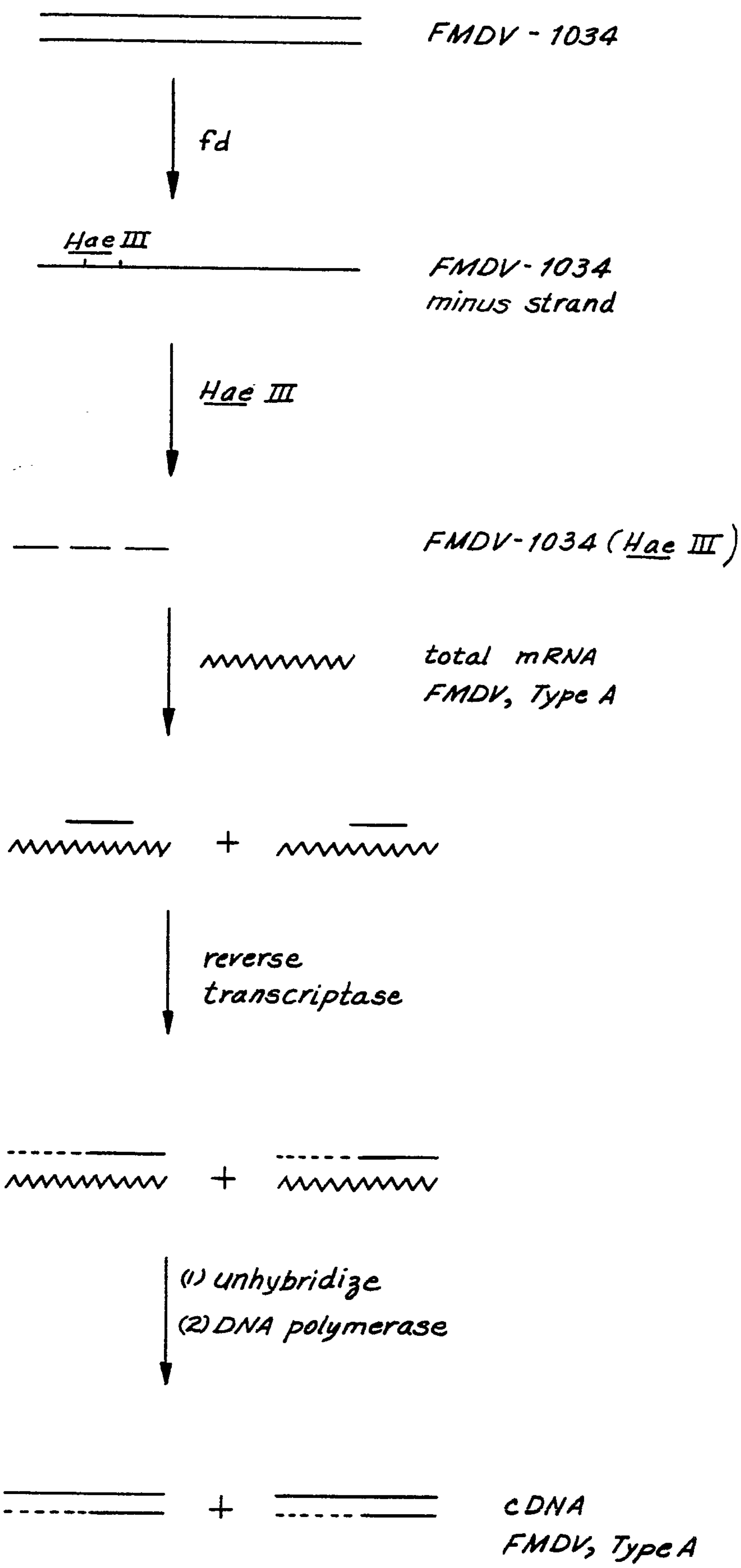
FMDV - 1034
fd
Hae III
FMDV-1034
minus strand
Hae III
FMDV-1034 (Hae III)
total mRNA
FMDV, Type A
+
reverse
transcriptase
+
(1) unhybridize
(2) DNA polymerase
+
cDNA
FMDV, Type A

European Patent Office

**PARTIAL EUROPEAN SEARCH REPORT**
which under Rule 45 of the European Patent Convention shall be considered, for the purposes of subsequent proceedings, as the European search report

0040922

Application number
EP 81 30 2080

## DOCUMENTS CONSIDERED TO BE RELEVANT

| Category | Citation of document with indication, where appropriate, of relevant passages | Relevant to claim | CLASSIFICATION OF THE APPLICATION (Int. Cl.³) |
|---|---|---|---|
| | NATURE, vol.276, 16th November 1978 Mc.Millan Journals Ltd. A.G. PORTER et al.: "3'-Terminal nucleotide sequences in the genome RNA of picornaviruses", pages 298-301 * Figures 2,3 * -- | 1 | C 12 N 15/00<br>C 12 P 21/02<br>C 07 H 21/04<br>C 12 N 1/20<br>C 07 C 103/52//<br>C 12 R 1/19<br>1/38<br>1/07 |
| PX | NATURE, vol. 289, 12th February 1981, Mc.Millan Journals Ltd. H. KUPPER et al.: "Cloning of cDNA of major antigen of foot and mouth disease virus and expression in E.coli", pages 555-559, no. 5798 Chesham Bucks, GB * The whole document * -- | 1-39 | TECHNICAL FIELDS SEARCHED (Int. Cl.³) |
| PX | CHEMICAL ABSTRACTS, vol. 94, 1981 2nd February, no. 5, page 182 abstract 26318a Columbus, Ohio, US I.N. TITOV et al.: "Enzymic synthesis and properties of DNA complementary to aphthosa virus RNA" & Biokhimiya (Moscow) 1980, 45(11), 2059-64 -- ./. | 1-8 | C 12 N 15/00<br>C 12 P 21/02<br>C 07 H 21/04<br>C 12 N 1/20<br>C 07 C 103/52<br>C 12 R 1/19<br>1/38//<br>C 12 R 1/07<br>1/125 |

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-39

Claims searched incompletely:

Claims not searched: 40

Reason for the limitation of the search: Method for treatment of the human or animal body by surgery or therapy (see article 52(4) of the European Patent Convention).

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

| Place of search | Date of completion of the search | Examiner |
|---|---|---|
| The Hague | 26-08-1981 | DESCAMPS |

European Patent Office

**PARTIAL EUROPEAN SEARCH REPORT**

0040922

Application number EP 81 30 2080



| DOCUMENTS CONSIDERED TO BE RELEVANT | | | CLASSIFICATION OF THE APPLICATION (Int. Cl.³) |
|---|---|---|---|
| Category | Citation of document with indication, where appropriate, of relevant passages | Relevant to claim | |
| A | CHEMICAL ABSTRACTS, vol. 92, 28th April 1980, no. 17, page 165, abstract 141900e Columbus, Ohio, US D.V. SANGAR et al.: "Location of the initiation site for protein synthesis on foot-and-mouth disease virus RNA by in vitro translation of defined fragments of the RNA" J. Virol. 1980, 33(1), 59-68 ---- | | |
| | | | TECHNICAL FIELDS SEARCHED (Int. Cl.³) |